(19) Europäisches Patentamt
European Patent Office
Office européen des brevets

(11) **EP 4 796 637 A1**

(12) **EUROPEAN PATENT APPLICATION**
published in accordance with Art. 153(4) EPC

(43) Date of publication:
**26.08.2026 Bulletin 2026/35**

(21) Application number: **24879019.8**

(22) Date of filing: **16.10.2024**

(51) International Patent Classification (IPC):
***C12N 15/13*** *(2006.01)* ***A61K 39/395*** *(2006.01)*
***A61K 9/19*** *(2006.01)* ***A61K 47/18*** *(2017.01)*
***A61K 47/10*** *(2017.01)* ***A61P 35/00*** *(2006.01)*
***A61P 37/00*** *(2006.01)*

(52) Cooperative Patent Classification (CPC):
**A61K 9/19; A61K 39/395; A61K 47/10; A61K 47/18; A61P 35/00; A61P 37/00; C07K 16/00**

(86) International application number:
**PCT/CN2024/125181**

(87) International publication number:
**WO 2025/082377 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.10.2023 CN 202311342148**
**24.07.2024 CN 202410995421**

(71) Applicants:
- **Jiangsu Hengrui Pharmaceuticals Co., Ltd.**
**Lianyungang, Jiangsu 222047 (CN)**
- **Shanghai Hengrui Pharmaceutical Co., Ltd.**
**Shanghai 200245 (CN)**

(72) Inventors:
- **SU, Yan**
**Shanghai 200245 (CN)**
- **WU, Tingting**
**Shanghai 200245 (CN)**

(74) Representative: **Broom, Ashley John**
**GSK**
**Legal & Compliance - Global Patents**
**79 New Oxford Street**
**London WC1A 1DG (GB)**

Remarks:
The complete document including Reference Table(s) and the Sequence Listing(s) can be downloaded from the EPO website

# (54) PHARMACEUTICAL COMPOSITION COMPRISING ANTI-TSLP ANTIBODY AND USE THEREOF

(57) The present disclosure relates to an anti-TSLP antibody pharmaceutical composition and uses thereof. Specifically, the disclosure relates to a pharmaceutical composition comprising an anti-TSLP antibody, a surfactant and excipients, and uses thereof for treating TSLP-related diseases or conditions.



Processed by Luminess, 75001 PARIS (FR)

## Description

The present application claims priority to Chinese Patent Application CN202311342148.9 filed on 17 October 2023 and Chinese Patent Application CN202410995421.6 filed on 24 July 2024.

## Technical Field

The present disclosure belongs to the field of pharmaceutical formulations, specifically relates to a pharmaceutical composition comprising an anti-TSLP antibody, and uses thereof as a medicament.

## Background Art

The statements herein only provide background information related to the present disclosure and do not necessarily constitute prior art.

Asthma is a serious chronic inflammatory disease of the airway, affecting approximately 334 million people worldwide. With environmental degradation and increased air pollution, more people may suffer from this disease, posing a serious threat to human life and health.

Thymic stromal lymphopoietin (TSLP) is a cytokine produced by epithelial cells in response to pro-inflammatory stimuli, and mainly promote allergic inflammatory responses via its action on dendritic cells and mast cells. TSLP is a cytokine similar to interleukin-7 (IL-7) and was first discovered in conditioned media from murine thymic stromal cells. TSLP is predominantly expressed in the epithelial cells of the lungs, skin and intestines. TSLP consists of four alpha-helices and two loops, AB and CD, with three pairs of disulphide bonds formed by six cysteine residues, two N-glycosylation sites, and a molecular weight of approximately 15-20 kD. The TSLP receptor is a complex comprising two components: TSLPR and IL-7Rα. TSLP initially binds to TSLPR with relatively low affinity, then recruits IL-7Rα with high affinity, ultimately activating signalling pathways such as STAT5, leading to the maturation of dendritic cells (DCs) and the differentiation of T cells.

Myeloid dendritic cells (mDCs) are the primary effector cells of TSLP; TSLP acts on immature mDCs, inducing the secretion of cytokines IL-8, eotaxin-2, TARC and MDC, and upregulating OX40L expression. In the absence of IL-12, OX40L binds to naive $CD4^+$ T cells, promoting their differentiation into Th2 cells, which subsequently secrete Th2 cytokines such as IL-5, IL-4, IL-9, IL-13, and TNF, thus inducing Th2-mediated inflammatory responses. Additionally, TSLP can induce DCs to produce IL-8, which recruits neutrophils, resulting in innate immune inflammation mediated by neutrophils. TSLP can also induce DCs to produce eotaxin-2, which recruits eosinophils; together with IL-5, this facilitates rapid eosinophilic infiltration and inflammation. TSLP also acts on mast cells and natural killer cells, mediating innate inflammation through the induction of IL-4, IL-6, IgE and other factors. In summary, TSLP can simultaneously induce both innate and Th2 inflammation, leading to increased mucus production, airway remodelling and narrowing, severe cellular fibrosis, and progressive development into three major allergic diseases, i.e., asthma, atopic dermatitis, and allergic rhinitis. Therefore, blocking TSLP is a potentially effective strategy for treating asthma, atopic dermatitis and related diseases.

WO2020244544A1 has disclosed the use of anti-TSLP antibodies for treating allergic diseases, cancers and immune diseases, and meanwhile WO2022116858A1 has disclosed an anti-TSLP antibody pharmaceutical composition and uses thereof. In the biopharmaceutical field, polysorbates are the most commonly used surfactants, but may undergo degradation, lose efficacy, or even form particles when influenced by factors such as chemical or enzymatic hydrolysis and oxidation, wherein polysorbate 80 is more susceptible to oxidation-induced degradation than polysorbate 20 (Johanna Weber, et al. International journal of pharmaceutics: X, 2023, 27(6): 100202.) Therefore, in order to better exert the therapeutic effect of antibodies, research on stable formulations of antibody drugs is particularly important.

## Summary of the Invention

The present disclosure provides a pharmaceutical composition comprising an anti-TSLP antibody. The pharmaceutical composition has advantages such as good stability.

In some embodiments, the present disclosure provides a pharmaceutical composition comprising an anti-TSLP antibody, a surfactant and excipients, wherein the anti-TSLP antibody comprises a heavy chain variable region and a light chain variable region, wherein the heavy chain variable region comprises HCDR1 as shown in SEQ ID NO: 1, HCDR2 as shown in SEQ ID NO: 2, and HCDR3 as shown in SEQ ID NO: 3; and the light chain variable region comprises LCDR1 as shown in SEQ ID NO: 4, LCDR2 as shown in SEQ ID NO: 5, and LCDR3 as shown in SEQ ID NO: 6.

In some embodiments, the pharmaceutical composition as described above comprises an anti-TSLP antibody, poloxamer and excipients, wherein the anti-TSLP antibody comprises a heavy chain variable region and a light chain variable region, wherein the heavy chain variable region comprises HCDR1 as shown in SEQ ID NO: 1, HCDR2 as shown

in SEQ ID NO: 2, and HCDR3 as shown in SEQ ID NO: 3; and the light chain variable region comprises LCDR1 as shown in SEQ ID NO: 4, LCDR2 as shown in SEQ ID NO: 5, and LCDR3 as shown in SEQ ID NO: 6.

In some embodiments, the pharmaceutical composition as described above comprises an anti-TSLP antibody, a surfactant and excipients, wherein the surfactant is poloxamer, and the anti-TSLP antibody comprises a heavy chain variable region and a light chain variable region, wherein the heavy chain variable region comprises HCDR1 as shown in SEQ ID NO: 1, HCDR2 as shown in SEQ ID NO: 2, and HCDR3 as shown in SEQ ID NO: 3; and the light chain variable region comprises LCDR1 as shown in SEQ ID NO: 4, LCDR2 as shown in SEQ ID NO: 5, and LCDR3 as shown in SEQ ID NO: 6.

In some embodiments, in the pharmaceutical composition according to any one of the above, in the anti-TSLP antibody, the HCDR1, HCDR2, HCDR3, LCDR1, LCDR2 and LCDR3 are defined according to Kabat, IMGT, Chothia, AbM or Contact numbering schemes; preferably defined according to the Kabat numbering scheme.

In some embodiments, in the pharmaceutical composition according to any one of the above, the amino acid sequence of the heavy chain variable region of the anti-TSLP antibody has at least 85% (for example 85%, 86%, 87%, 88%, 89%, 90%, 91%, 92%, 93%, 94%, 95%, 96%, 97%, 98%, 99% or 100%) sequence identity with the heavy chain variable region of the hu179-33 antibody (SEQ ID NO: 7), and the amino acid sequence of the light chain variable region of the anti-TSLP antibody has at least 85% (for example 85%, 86%, 87%, 88%, 89%, 90%, 91%, 92%, 93%, 94%, 95%, 96%, 97%, 98%, 99% or 100%) sequence identity with the light chain variable region of the hu179-33 antibody (SEQ ID NO: 8). In some embodiments, the heavy chain variable region of the anti-TSLP antibody comprises the amino acid sequence as shown in SEQ ID NO: 7, and the light chain variable region comprises the amino acid sequence as shown in SEQ ID NO: 8.

In some embodiments, in the pharmaceutical composition according to any one of the above, the anti-TSLP antibody comprises a heavy chain constant region and a light chain constant region, wherein the heavy chain constant region of the anti-TSLP antibody comprises the amino acid sequence as shown in SEQ ID NO: 9, and the light chain constant region comprises the amino acid sequence as shown in SEQ ID NO: 10.

In some embodiments, in the pharmaceutical composition according to any one of the above, the anti-TSLP antibody comprises a heavy chain and a light chain, wherein the amino acid sequence of the heavy chain of the anti-TSLP antibody has at least 85% (for example 85%, 86%, 87%, 88%, 89%, 90%, 91%, 92%, 93%, 94%, 95%, 96%, 97%, 98%, 99% or 100%) sequence identity with the heavy chain of the hu179-33 antibody (SEQ ID NO: 11), and the amino acid sequence of the light chain of the anti-TSLP antibody has at least 85% (for example 85%, 86%, 87%, 88%, 89%, 90%, 91%, 92%, 93%, 94%, 95%, 96%, 97%, 98%, 99% or 100%) sequence identity with the light chain of the hu179-33 antibody (SEQ ID NO: 12). In some embodiments, the anti-TSLP antibody comprises a heavy chain as shown in SEQ ID NO: 11, and a light chain as shown in SEQ ID NO: 12.

In some embodiments, the anti-TSLP antibody comprises a heavy chain and a light chain, wherein the amino acid sequence of the heavy chain is as shown in SEQ ID NO: 11, and the amino acid sequence of the light chain is as shown in SEQ ID NO: 12, or

the amino acid sequence of the heavy chain is as shown in amino acid residues 1 to 448 of SEQ ID NO: 11, and the amino acid sequence of the light chain is as shown in amino acid residues 1 to 214 of SEQ ID NO: 12.

In some embodiments, in the pharmaceutical composition according to any one of the above, the surfactant is a non-ionic surfactant. In some embodiments, the surfactant is selected from poloxamers (for example poloxamer 188), polysorbates (for example polysorbate 20, polysorbate 80), Triton, sodium dodecyl sulphate, sodium lauryl sulphate, sodium octyl glucoside, lauryl sulphobetaine, myristyl sulphobetaine, linoleyl sulphobetaine, stearyl sulphobetaine, lauroyl sarcosinate, myristoyl sarcosinate, linoleoyl sarcosinate, stearoyl sarcosinate, linoleyl betaine, myristyl betaine, cetyl betaine, lauramidopropyl betaine, cocamidopropyl betaine, linoleamidopropyl betaine, myristamidopropyl betaine, palmitamidopropyl betaine, isostearamidopropyl betaine, myristamidopropyl dimethylamine, palmitamidopropyl dimethylamine, isostearamidopropyl dimethylamine, sodium methyl cocoyl taurate, sodium methyl oleoyl taurate, polythene glycol, polypropylene glycol, copolymers of ethylene glycol and propylene glycol, and the like. In some embodiments, the surfactant is poloxamer or polysorbate. In some embodiments, the surfactant is poloxamer 188 or polysorbate 80. In some embodiments, the surfactant is poloxamer 188.

In some embodiments, in the pharmaceutical composition according to any one of the above, the excipients are selected from magnesium chloride, calcium chloride, sodium chloride, sodium fluoride, sodium thiocyanate, potassium chloride, sodium acetate, sodium sulphate, sodium iodide, arginine (including arginine hydrochloride), proline, histidine and lysine. In some embodiments, the excipient is an amino acid, such as an L- or D-amino acid, for example L-arginine, D-arginine, L-lysine or L-histidine. In some embodiments, the excipient comprises a salt form, for example a salt of arginine, lysine or histidine. In some embodiments, the excipient is selected from arginine hydrochloride, proline, histidine hydrochloride, lysine hydrochloride, calcium chloride, magnesium chloride and sodium chloride. In some embodiments, the excipient is arginine hydrochloride or proline. In some embodiments, the excipient is arginine hydrochloride. In some embodiments, the excipient is L-arginine hydrochloride.

In some embodiments, in the pharmaceutical composition according to any one of the above, the anti-TSLP antibody has a concentration from 1 mg/mL to 250 mg/mL. In some embodiments, the anti-TSLP antibody has a

concentration from 90 mg/mL to 200 mg/mL. In some embodiments, the anti-TSLP antibody has a concentration from 150 mg/mL to 200 mg/mL. In some embodiments, the anti-TSLP antibody has a concentration from 180 mg/mL to 200 mg/mL. In some embodiments, the anti-TSLP antibody has a concentration from 145.6 mg/mL to 218.4 mg/mL. In some embodiments, the anti-TSLP antibody has a concentration from 163.8 mg/mL to 200.2 mg/mL. In some embodiments, the anti-TSLP antibody has a concentration from 172.9 mg/mL to 191.1 mg/mL. In some embodiments, the anti-TSLP antibody has a concentration of about 90 mg/mL. In some embodiments, the anti-TSLP antibody has a concentration of about 100 mg/mL. In some embodiments, the anti-TSLP antibody has a concentration of about 150 mg/mL. In some embodiments, the anti-TSLP antibody has a concentration of about 180 mg/mL. In some embodiments, the anti-TSLP antibody has a concentration of about 182 mg/mL. In some embodiments, the anti-TSLP antibody has a concentration of about 200 mg/mL. In some embodiments, the anti-TSLP antibody has a concentration of about 1 mg/mL, about 5 mg/mL, about 10 mg/mL, about 20 mg/mL, about 30 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 105 mg/mL, about 110 mg/mL, about 120 mg/mL, about 130 mg/mL, about 140 mg/mL, about 145.6 mg/mL, about 150 mg/mL, about 160 mg/mL, about 163.8 mg/mL, about 170 mg/mL, about 172.9 mg/mL, about 175 mg/mL, about 180 mg/mL, about 181 mg/mL, about 182 mg/mL, about 183 mg/mL, about 184 mg/mL, about 185 mg/mL, about 190 mg/mL, about 191.1 mg/mL, about 200 mg/mL, about 200.2 mg/mL, about 210 mg/mL, about 218.4 mg/mL, about 220 mg/mL, about 230 mg/mL, about 240 mg/mL or about 250 mg/mL. In some embodiments, the anti-TSLP antibody has a concentration of 1 mg/mL, 5 mg/mL, 10 mg/mL, 20 mg/mL, 30 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 120 mg/mL, 130 mg/mL, 140 mg/mL, 145.6 mg/mL, 150 mg/mL, 160 mg/mL, 163.8 mg/mL, 170 mg/mL, 172.9 mg/mL, 175 mg/mL, 180 mg/mL, 181 mg/mL, 182 mg/mL, 183 mg/mL, 184 mg/mL, 185 mg/mL, 190 mg/mL, 191.1 mg/mL, 200 mg/mL, 200.2 mg/mL, 210 mg/mL, 218.4 mg/mL, 220 mg/mL, 230 mg/mL, 240 mg/mL or 250 mg/mL, or any range between these point values. In some embodiments, the anti-TSLP antibody has a concentration of 90 mg/mL. In some embodiments, the anti-TSLP antibody has a concentration of 100 mg/mL. In some embodiments, the anti-TSLP antibody has a concentration of 150 mg/mL. In some embodiments, the anti-TSLP antibody has a concentration of 180 mg/mL. In some embodiments, the anti-TSLP antibody has a concentration of 182 mg/mL. In some embodiments, the anti-TSLP antibody has a concentration of 200 mg/mL.

In some embodiments, in the pharmaceutical composition according to any one of the above, the surfactant has a concentration from 0.01 mg/mL to 2 mg/mL. In some embodiments, the surfactant has a concentration from 0.2 mg/mL to 2 mg/mL. In some embodiments, the surfactant has a concentration from 0.2 mg/mL to 1 mg/mL. In some embodiments, the surfactant has a concentration from 0.2 mg/mL to 0.8 mg/mL. In some embodiments, the surfactant has a concentration from 0.64 mg/mL to 0.96 mg/mL. In some embodiments, the surfactant has a concentration from 0.72 mg/mL to 0.88 mg/mL. In some embodiments, the surfactant has a concentration from 0.76 mg/mL to 0.84 mg/mL. In some embodiments, the surfactant has a concentration of about 0.2 mg/mL. In some embodiments, the surfactant has a concentration of about 0.8 mg/mL. In some embodiments, the surfactant concentration is about 0.01 mg/mL, about 0.05 mg/mL, about 0.1 mg/mL, about 0.15 mg/mL, about 0.2 mg/mL, about 0.3 mg/mL, about 0.4 mg/mL, about 0.5 mg/mL, about 0.6 mg/mL, about 0.64 mg/mL, about 0.7 mg/mL, about 0.72 mg/mL, about 0.75 mg/mL, about 0.76 mg/mL, about 0.8 mg/mL, about 0.84 mg/mL, about 0.85 mg/mL, about 0.88 mg/mL, about 0.9 mg/mL, about 0.96 mg/mL, about 1.0 mg/mL, about 1.1 mg/mL, about 1.2 mg/mL, about 1.3 mg/mL, about 1.4 mg/mL, about 1.5 mg/mL, about 1.6 mg/mL, about 1.7 mg/mL, about 1.8 mg/mL, about 1.9 mg/mL or about 2.0 mg/mL. In some embodiments, the surfactant concentration is 0.01 mg/mL, 0.05 mg/mL, 0.1 mg/mL, 0.15 mg/mL, 0.2 mg/mL, 0.3 mg/mL, 0.4 mg/mL, 0.5 mg/mL, 0.6 mg/mL, 0.64 mg/mL, 0.7 mg/mL, 0.72 mg/mL, 0.75 mg/mL, 0.76 mg/mL, 0.8 mg/mL, 0.84 mg/mL, 0.85 mg/mL, 0.88 mg/mL, 0.9 mg/mL, 0.96 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL or 2.0 mg/mL, or any range between these point values. In some embodiments, the surfactant has a concentration of 0.2 mg/mL. In some embodiments, the surfactant has a concentration of 0.8 mg/mL.

In some embodiments, in the pharmaceutical composition according to any one of the above, the poloxamer 188 has a concentration from 0.01 mg/mL to 2 mg/mL. In some embodiments, the poloxamer 188 has a concentration from 0.2 mg/mL to 2 mg/mL. In some embodiments, the poloxamer 188 has a concentration from 0.2 mg/mL to 1 mg/mL. In some embodiments, the poloxamer 188 has a concentration from 0.2 mg/mL to 0.8 mg/mL. In some embodiments, the poloxamer 188 has a concentration from 0.64 mg/mL to 0.96 mg/mL. In some embodiments, the poloxamer 188 has a concentration from 0.72 mg/mL to 0.88 mg/mL. In some embodiments, the poloxamer 188 has a concentration from 0.76 mg/mL to 0.84 mg/mL. In some embodiments, the poloxamer 188 has a concentration of about 0.2 mg/mL. In some embodiments, the poloxamer 188 has a concentration of about 0.8 mg/mL. In some embodiments, the poloxamer 188 has a concentration of about 0.01 mg/mL, about 0.05 mg/mL, about 0.1 mg/mL, about 0.15 mg/mL, about 0.2 mg/mL, about 0.3 mg/mL, about 0.4 mg/mL, about 0.5 mg/mL, about 0.6 mg/mL, about 0.64 mg/mL, about 0.7 mg/mL, about 0.72 mg/mL, about 0.75 mg/mL, about 0.76 mg/mL, about 0.8 mg/mL, about 0.84 mg/mL, about 0.85 mg/mL, about 0.88 mg/mL, about 0.9 mg/mL, about 0.96 mg/mL, about 1.0 mg/mL, about 1.1 mg/mL, about 1.2 mg/mL, about 1.3 mg/mL, about 1.4 mg/mL, about 1.5 mg/mL, about 1.6 mg/mL, about 1.7 mg/mL, about 1.8 mg/mL, about 1.9 mg/mL or about 2.0 mg/mL. In some

embodiments, the poloxamer 188 has a concentration of 0.01 mg/mL, 0.05 mg/mL, 0.1 mg/mL, 0.15 mg/mL, 0.2 mg/mL, 0.3 mg/mL, 0.4 mg/mL, 0.5 mg/mL, 0.6 mg/mL, 0.64 mg/mL, 0.7 mg/mL, 0.72 mg/mL, 0.75 mg/mL, 0.76 mg/mL, 0.8 mg/mL, 0.84 mg/mL, 0.85 mg/mL, 0.88 mg/mL, 0.9 mg/mL, 0.96 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL or 2.0 mg/mL, or any range between these point values. In some embodiments, the poloxamer 188 has a concentration of 0.2 mg/mL. In some embodiments, the poloxamer 188 has a concentration of 0.8 mg/mL.

In some embodiments, in the pharmaceutical composition according to any one of the above, the excipient has a concentration from 1 mM to 300 mM. In some embodiments, the excipient has a concentration from 50 mM to 200 mM. In some embodiments, the excipient has a concentration from 50 mM to 150 mM. In some embodiments, the excipient has a concentration from 50 mM to 125 mM. In some embodiments, the excipient has a concentration from 95 mM to 150 mM. In some embodiments, the excipient has a concentration from 100 mM to 150 mM. In some embodiments, the excipient has a concentration from 112.5 mM to 137.5 mM. In some embodiments, the excipient has a concentration from 118.75 mM to 131.25 mM. In some embodiments, the excipient has a concentration of about 125 mM. In some embodiments, the excipient has a concentration from 88 mM to 132 mM. In some embodiments, the excipient has a concentration from 99 mM to 121 mM. In some embodiments, the excipient has a concentration from 104.5 mM to 115.5 mM. In some embodiments, the excipient has a concentration of about 110 mM. In some embodiments, the excipient has a concentration of about 50 mM. In some embodiments, the excipient has a concentration of about 150 mM. In some embodiments, the excipient has a concentration of about 1 mM, about 5 mM, about 10 mM, about 20 mM, about 30 mM, about 40 mM, about 45 mM, about 50 mM, about 55 mM, about 60 mM, about 65 mM, about 70 mM, about 75 mM, about 80 mM, about 85 mM, about 88 mM, about 90 mM, about 95 mM, about 99 mM, about 100 mM, about 104.5 mM, about 105 mM, about 110 mM, about 112.5 mM, about 115 mM, about 115.5 mM, about 118.75 mM, about 120 mM, about 121 mM, about 125 mM, about 130 mM, about 131.25 mM, about 132 mM, about 137.5 mM, about 140 mM, about 150 mM, about 160 mM, about 170 mM, about 175 mM, about 180 mM, about 190 mM, about 200 mM, about 210 mM, about 220 mM, about 230 mM, about 240 mM, about 250 mM, about 260 mM, about 270 mM, about 280 mM, about 290 mM or about 300 mM. In some embodiments, the excipient has a concentration of 1 mM, 5 mM, 10 mM, 20 mM, 30 mM, 40 mM, 45 mM, 50 mM, 55 mM, 60 mM, 65 mM, 70 mM, 75 mM, 80 mM, 85 mM, 88 mM, 90 mM, 95 mM, 99 mM, 100 mM, 104.5 mM, 105 mM, 110 mM, 112.5 mM, 115 mM, 115.5 mM, 118.75 mM, 120 mM, 121 mM, 125 mM, 130 mM, 131.25 mM, 132 mM, 137.5 mM, 140 mM, 150 mM, 160 mM, 170 mM, 175 mM, 180 mM, 190 mM, 200 mM, 210 mM, 220 mM, 230 mM, 240 mM, 250 mM, 260 mM, 270 mM, 280 mM, 290 mM or 300 mM, or any range between these point values. In some embodiments, the excipient has a concentration of 125 mM. In some embodiments, the excipient has a concentration of 110 mM. In some embodiments, the excipient has a concentration of 50 mM. In some embodiments, the excipient has a concentration of 150 mM.

In some embodiments, in the pharmaceutical composition according to any one of the above, the arginine hydrochloride has a concentration from 1 mM to 300 mM. In some embodiments, the arginine hydrochloride has a concentration from 50 mM to 200 mM. In some embodiments, the arginine hydrochloride has a concentration from 50 mM to 150 mM. In some embodiments, the arginine hydrochloride has a concentration from 50 mM to 125 mM. In some embodiments, the arginine hydrochloride has a concentration from 95 mM to 150 mM. In some embodiments, the arginine hydrochloride has a concentration from 100 mM to 150 mM. In some embodiments, the arginine hydrochloride has a concentration from 112.5 mM to 137.5 mM. In some embodiments, the arginine hydrochloride has a concentration from 118.75 mM to 131.25 mM. In some embodiments, the arginine hydrochloride has a concentration of about 125 mM. In some embodiments, the arginine hydrochloride has a concentration from 88 mM to 132 mM. In some embodiments, the arginine hydrochloride has a concentration from 99 mM to 121 mM. In some embodiments, the arginine hydrochloride has a concentration from 104.5 mM to 115.5 mM. In some embodiments, the arginine hydrochloride has a concentration of about 110 mM. In some embodiments, the arginine hydrochloride has a concentration of about 50 mM. In some embodiments, the arginine hydrochloride has a concentration of about 150 mM. In some embodiments, the arginine hydrochloride has a concentration of about 1 mM, about 5 mM, about 10 mM, about 20 mM, about 30 mM, about 40 mM, about 45 mM, about 50 mM, about 55 mM, about 60 mM, about 65 mM, about 70 mM, about 75 mM, about 80 mM, about 85 mM, about 88 mM, about 90 mM, about 95 mM, about 99 mM, about 100 mM, about 104.5 mM, about 105 mM, about 110 mM, about 112.5 mM, about 115 mM, about 115.5 mM, about 118.75 mM, about 120 mM, about 121 mM, about 125 mM, about 130 mM, about 131.25 mM, about 132 mM, about 137.5 mM, about 140 mM, about 150 mM, about 160 mM, about 170 mM, about 175 mM, about 180 mM, about 190 mM, about 200 mM, about 210 mM, about 220 mM, about 230 mM, about 240 mM, about 250 mM, about 260 mM, about 270 mM, about 280 mM, about 290 mM or about 300 mM. In some embodiments, the arginine hydrochloride has a concentration of 1 mM, 5 mM, 10 mM, 20 mM, 30 mM, 40 mM, 45 mM, 50 mM, 55 mM, 60 mM, 65 mM, 70 mM, 75 mM, 80 mM, 85 mM, 88 mM, 90 mM, 95 mM, 99 mM, 100 mM, 104.5 mM, 105 mM, 110 mM, 112.5 mM, 115 mM, 115.5 mM, 118.75 mM, 120 mM, 121 mM, 125 mM, 130 mM, 131.25 mM, 132 mM, 137.5 mM, 140 mM, 150 mM, 160 mM, 170 mM, 175 mM, 180 mM, 190 mM, 200 mM, 210 mM, 220 mM, 230 mM, 240 mM, 250 mM, 260 mM, 270 mM, 280 mM, 290 mM or 300 mM, or any range between these point values. In some embodiments, the arginine hydrochloride has a concentration of 125 mM. In some embodiments, the arginine hydrochloride has a concentration of 110 mM. In some embodiments, the arginine hydrochloride has a concentration of 50 mM. In some

embodiments, the arginine hydrochloride has a concentration of 150 mM.

In some embodiments, in the pharmaceutical composition according to any one of the above, the pharmaceutical composition further comprises a buffer. In some embodiments, the buffer is a histidinate buffer, an acetate buffer, a citrate buffer, a succinate buffer or a phosphate buffer. In some embodiments, the buffer is a histidinate buffer. In some embodiments, the buffer is a histidine-acetate histidine buffer.

In some embodiments, in the pharmaceutical composition according to any one of the above, the buffer has a concentration from 5 mM to 100 mM. In some embodiments, the buffer has a concentration from 10 mM to 60 mM. In some embodiments, the buffer has a concentration from 35 mM to 55 mM. In some embodiments, the buffer has a concentration from 10 mM to 50 mM. In some embodiments, the buffer has a concentration from 20 mM to 50 mM. In some embodiments, the buffer has a concentration from 30 mM to 50 mM. In some embodiments, the buffer has a concentration from 32 mM to 48 mM. In some embodiments, the buffer has a concentration from 40 mM to 60 mM. In some embodiments, the buffer has a concentration from 45 mM to 55 mM. In some embodiments, the buffer has a concentration from 47.5 mM to 52.5 mM. In some embodiments, the buffer has a concentration of about 60 mM. In some embodiments, the buffer has a concentration of about 50 mM. In some embodiments, the buffer has a concentration from 36 mM to 44 mM. In some embodiments, the buffer has a concentration from 38 mM to 42 mM. In some embodiments, the buffer has a concentration of about 40 mM. In some embodiments, the buffer has a concentration of about 30 mM. In some embodiments, the buffer has a concentration of about 20 mM. In some embodiments, the buffer has a concentration of about 10 mM. In some embodiments, the buffer has a concentration of about 5 mM, about 10 mM, about 20 mM, about 30 mM, about 32 mM, about 35 mM, about 36 mM, about 38 mM, about 40 mM, about 42 mM, about 44 mM, about 45 mM, about 47.5 mM, about 48 mM, about 50 mM, about 52.5 mM, about 55 mM, about 60 mM, about 65 mM, about 70 mM, about 75 mM, about 80 mM, about 85 mM, about 90 mM, about 95 mM or about 100 mM. In some embodiments, the buffer has a concentration of 5 mM, 10 mM, 20 mM, 30 mM, 32 mM, 35 mM, 36 mM, 38 mM, 40 mM, 42 mM, 44 mM, 45 mM, 47.5 mM, 48 mM, 50 mM, 52.5 mM, 55 mM, 60 mM, 65 mM, 70 mM, 75 mM, 80 mM, 85 mM, 90 mM, 95 mM or 100 mM, or any range between these point values. In some embodiments, the buffer has a concentration of 60 mM. In some embodiments, the buffer has a concentration of 50 mM. In some embodiments, the buffer has a concentration of 40 mM. In some embodiments, the buffer has a concentration of 30 mM. In some embodiments, the buffer has a concentration of 20 mM. In some embodiments, the buffer has a concentration of 10 mM.

In some embodiments, in the pharmaceutical composition according to any one of the above, the histidine-acetate histidine buffer has a concentration from 5 mM to 100 mM. In some embodiments, the histidine-acetate histidine buffer has a concentration from 10 mM to 60 mM. In some embodiments, the histidine-acetate histidine buffer has a concentration from 35 mM to 55 mM. In some embodiments, the histidine-acetate histidine buffer has a concentration from 10 mM to 50 mM. In some embodiments, the histidine-acetate histidine buffer has a concentration from 20 mM to 50 mM. In some embodiments, the histidine-acetate histidine buffer has a concentration from 30 mM to 50 mM. In some embodiments, the histidine-acetate histidine buffer has a concentration from 32 mM to 48 mM. In some embodiments, the histidine-acetate histidine buffer has a concentration from 40 mM to 60 mM. In some embodiments, the histidine-acetate histidine buffer has a concentration from 45 mM to 55 mM. In some embodiments, the histidine-acetate histidine buffer has a concentration from 47.5 mM to 52.5 mM. In some embodiments, the histidine-acetate histidine buffer has a concentration of about 60 mM. In some embodiments, the histidine-acetate histidine buffer has a concentration of about 50 mM. In some embodiments, the histidine-acetate histidine buffer has a concentration from 36 mM to 44 mM. In some embodiments, the histidine-acetate histidine buffer has a concentration from 38 mM to 42 mM. In some embodiments, the histidine-acetate histidine buffer has a concentration of about 40 mM. In some embodiments, the histidine-acetate histidine buffer has a concentration of about 30 mM. In some embodiments, the histidine-acetate histidine buffer has a concentration of about 20 mM. In some embodiments, the histidine-acetate histidine buffer has a concentration of about 10 mM. In some embodiments, the histidine-acetate histidine buffer has a concentration of about 5 mM, about 10 mM, about 20 mM, about 30 mM, about 32 mM, about 35 mM, about 36 mM, about 38 mM, about 40 mM, about 42 mM, about 44 mM, about 45 mM, about 47.5 mM, about 48 mM, about 50 mM, about 52.5 mM, about 55 mM, about 60 mM, about 65 mM, about 70 mM, about 75 mM, about 80 mM, about 85 mM, about 90 mM, about 95 mM or about 100 mM. In some embodiments, the histidine-acetate histidine buffer has a concentration of 5 mM, 10 mM, 20 mM, 30 mM, 32 mM, 35 mM, 36 mM, 38 mM, 40 mM, 42 mM, 44 mM, 45 mM, 47.5 mM, 48 mM, 50 mM, 52.5 mM, 55 mM, 60 mM, 65 mM, 70 mM, 75 mM, 80 mM, 85 mM, 90 mM, 95 mM or 100 mM, or any range between these point values. In some embodiments, the histidine-acetate histidine buffer has a concentration of 60 mM. In some embodiments, the histidine-acetate histidine buffer has a concentration of 50 mM. In some embodiments, the histidine-acetate histidine buffer has a concentration of 40 mM. In some embodiments, the histidine-acetate histidine buffer has a concentration of 30 mM. In some embodiments, the histidine-acetate histidine buffer has a concentration of 20 mM. In some embodiments, the histidine-acetate histidine buffer has a concentration of 10 mM.

In some embodiments, in the pharmaceutical composition according to any one of the above, the pH of the pharmaceutical composition is from 5.0 to 7.0. In some embodiments, the pH of the pharmaceutical composition is from 5.0 to 6.5. In some embodiments, the pH of the pharmaceutical composition is from 5.5 to 6.5. In some embodiments, the pH of

the pharmaceutical composition is from 5.7 to 6.3. In some embodiments, the pH of the pharmaceutical composition is from 5.8 to 6.2. In some embodiments, the pH of the pharmaceutical composition is about 6.0. In some embodiments, the pH of the pharmaceutical composition is about 5.0, about 5.5, about 5.6, about 5.7, about 5.8, about 5.9, about 6.0, about 6.1, about 6.2, about 6.3, about 6.4, about 6.5 or about 7.0. In some embodiments, the pH of the pharmaceutical composition is 5.0, 5.5, 5.6, 5.7, 5.8, 5.9, 6.0, 6.1, 6.2, 6.3, 6.4, 6.5 or 7.0, or any range between these point values. In some embodiments, the pH of the pharmaceutical composition is 5.0. In some embodiments, the pH of the pharmaceutical composition is 6.0. In some embodiments, the pH of the pharmaceutical composition is 6.5. When point values are referred to in the present disclosure, it should be understood that such point values include an error range. Such error range is caused by factors such as laboratory environment, operator handling, instruments, methodologies, and measurement errors. Taking pH as an example, when a measured value is about 6.0, it should be understood to include an error range. By way of example, when measuring a formulation using an industrial pH meter, "about 6.0" means $6.0 \pm 0.3$ (i.e., a pH from 5.7 to 6.3).

In some embodiments, the pharmaceutical composition according to any one of the above comprises:

(a) 1 mg/mL to 250 mg/mL of an anti-TSLP antibody,
(b) 0.01 mg/mL to 2 mg/mL of a surfactant,
(c) 1 mM to 300 mM of an excipient, and
(d) 5 mM to 100 mM of a buffer, wherein the pharmaceutical composition has a pH from 5.0 to 7.0.

In some embodiments, the pharmaceutical composition according to any one of the above comprises:

(a) 1 mg/mL to 250 mg/mL of an anti-TSLP antibody,
(b) 0.01 mg/mL to 2 mg/mL of poloxamer,
(c) 1 mM to 300 mM of an excipient, and
(d) 5 mM to 100 mM of a buffer, wherein the pharmaceutical composition has a pH from 5.0 to 7.0.

In some embodiments, the pharmaceutical composition according to any one of the above comprises:

(a) 90 mg/mL to 200 mg/mL of an anti-TSLP antibody,
(b) 0.2 mg/mL to 2 mg/mL of poloxamer 188,
(c) 50 mM to 200 mM of arginine hydrochloride or proline, and
(d) 10 mM to 60 mM of a histidine-acetate histidine buffer, wherein the pharmaceutical composition has a pH from 5.0 to 6.5.

In some embodiments, the pharmaceutical composition according to any one of the above comprises:

(a) 90 mg/mL to 200 mg/mL of an anti-TSLP antibody,
(b) 0.2 mg/mL to 2 mg/mL of poloxamer 188,
(c) 50 mM to 200 mM of arginine hydrochloride or proline, and
(d) 10 mM to 50 mM of a histidine-acetate histidine buffer, wherein the pharmaceutical composition has a pH from 5.5 to 6.5.

In some embodiments, the pharmaceutical composition according to any one of the above comprises:

(a) 150 mg/mL to 200 mg/mL of an anti-TSLP antibody,
(b) 0.2 mg/mL to 1 mg/mL of poloxamer 188,
(c) 50 mM to 150 mM of arginine hydrochloride, and
(d) 35 mM to 55 mM of a histidine-acetate histidine buffer, wherein the pharmaceutical composition has a pH from 5.0 to 6.5.

In some embodiments, the pharmaceutical composition according to any one of the above comprises:

(a) 150 mg/mL to 200 mg/mL of an anti-TSLP antibody,
(b) 0.2 mg/mL to 1 mg/mL of poloxamer 188,
(c) 50 mM to 150 mM of arginine hydrochloride, and
(d) 32 mM to 48 mM of a histidine-acetate histidine buffer, wherein the pharmaceutical composition has a pH from 5.7 to 6.3.

In some embodiments, the pharmaceutical composition according to any one of the above comprises:

(a) 150 mg/mL to 200 mg/mL of an anti-TSLP antibody,
(b) 0.64 mg/mL to 0.96 mg/mL of poloxamer 188,
(c) 95 mM to 150 mM of arginine hydrochloride, and
(d) 35 mM to 55 mM of a histidine-acetate histidine buffer, wherein the pharmaceutical composition has a pH from 5.7 to 6.3.

In some embodiments, the pharmaceutical composition according to any one of the above comprises:

(a) 180 mg/mL to 200 mg/mL of an anti-TSLP antibody,
(b) 0.64 mg/mL to 0.96 mg/mL of poloxamer 188,
(c) 95 mM to 150 mM of arginine hydrochloride, and
(d) 32 mM to 48 mM of a histidine-acetate histidine buffer, wherein the pharmaceutical composition has a pH from 5.7 to 6.3.

In some embodiments, the pharmaceutical composition according to any one of the above comprises:

(a) about 182 mg/mL of an anti-TSLP antibody,
(b) about 0.8 mg/mL of poloxamer 188,
(c) about 110 mM of arginine hydrochloride, and
(d) about 40 mM of a histidine-acetate histidine buffer, wherein the pharmaceutical composition has a pH of about 6.0.

In some embodiments, the pharmaceutical composition according to any one of the above comprises:

(a) about 182 mg/mL of an anti-TSLP antibody,
(b) about 0.8 mg/mL of poloxamer 188,
(c) about 125 mM of arginine hydrochloride, and
(d) about 50 mM of a histidine-acetate histidine buffer, wherein the pharmaceutical composition has a pH of about 6.0.
In some embodiments, the pharmaceutical composition according to any one of the above comprises:

(a) 150 mg/mL to 200 mg/mL of an anti-TSLP antibody comprising a heavy chain of SEQ ID NO: 11 and a light chain of SEQ ID NO: 12,
(b) 0.2 mg/mL to 1 mg/mL of poloxamer 188,
(c) 50 mM to 150 mM of arginine hydrochloride, and
(d) 32 mM to 48 mM of a histidine-acetate histidine buffer, wherein the pharmaceutical composition has a pH from 5.7 to 6.3.

In some embodiments, the pharmaceutical composition according to any one of the above comprises:

(a) 180 mg/mL to 200 mg/mL of an anti-TSLP antibody,
(b) 0.2 mg/mL to 0.8 mg/mL of poloxamer 188,
(c) 50 mM to 125 mM of arginine hydrochloride, and
(d) 30 mM to 50 mM of a histidine-acetate histidine buffer, wherein the pharmaceutical composition has a pH from 5.7 to 6.3.

In some embodiments, the pharmaceutical composition according to any one of the above comprises:

(a) 180 mg/mL to 200 mg/mL of an anti-TSLP antibody comprising a heavy chain of SEQ ID NO: 11 and a light chain of SEQ ID NO: 12,
(b) 0.2 mg/mL to 0.8 mg/mL of poloxamer 188,
(c) 50 mM to 125 mM of arginine hydrochloride, and
(d) 30 mM to 50 mM of a histidine-acetate histidine buffer, wherein the pharmaceutical composition has a pH from 5.7 to 6.3.

In some embodiments, the pharmaceutical composition according to any one of the above comprises:

(a) 180 mg/mL to 200 mg/mL of an anti-TSLP antibody comprising a heavy chain of SEQ ID NO: 11 and a light chain of SEQ ID NO: 12,
(b) 0.64 mg/mL to 0.96 mg/mL of poloxamer 188,

(c) 95 mM to 150 mM of arginine hydrochloride, and
(d) 32 mM to 48 mM of a histidine-acetate histidine buffer, wherein the pharmaceutical composition has a pH from 5.7 to 6.3.

In some embodiments, the pharmaceutical composition according to any one of the above comprises:

(a) about 182 mg/mL of an anti-TSLP antibody comprising a heavy chain of SEQ ID NO: 11 and a light chain of SEQ ID NO: 12,
(b) about 0.8 mg/mL of poloxamer 188,
(c) about 110 mM of arginine hydrochloride, and
(d) about 40 mM of a histidine-acetate histidine buffer, wherein the pharmaceutical composition has a pH of about 6.0.

In some embodiments, the pharmaceutical composition according to any one of the above comprises:

(a) 182 mg/mL of an anti-TSLP antibody comprising a heavy chain of SEQ ID NO: 11 and a light chain of SEQ ID NO: 12,
(b) 0.8 mg/mL of poloxamer 188,
(c) 110 mM of arginine hydrochloride, and
(d) 40 mM of a histidine-acetate histidine buffer, wherein the pharmaceutical composition has a pH of 6.0.

In some embodiments, the pharmaceutical composition according to any one of the above comprises:

(a) 150 mg/mL to 200 mg/mL of an anti-TSLP antibody comprising a heavy chain of SEQ ID NO: 11 and a light chain of SEQ ID NO: 12,
(b) 0.64 mg/mL to 0.96 mg/mL of poloxamer 188,
(c) 100 mM to 150 mM of arginine hydrochloride, and
(d) 40 mM to 60 mM of a histidine-acetate histidine buffer, wherein the pharmaceutical composition has a pH from 5.7 to 6.3.

In some embodiments, the pharmaceutical composition according to any one of the above comprises:

(a) about 182 mg/mL of an anti-TSLP antibody comprising a heavy chain of SEQ ID NO: 11 and a light chain of SEQ ID NO: 12,
(b) about 0.8 mg/mL of poloxamer 188,
(c) about 125 mM of arginine hydrochloride, and
(d) about 50 mM of a histidine-acetate histidine buffer, wherein the pharmaceutical composition has a pH from 5.7 to 6.3.

In some embodiments, the pharmaceutical composition according to any one of the above comprises:

(a) 182 mg/mL of an anti-TSLP antibody comprising a heavy chain of SEQ ID NO: 11 and a light chain of SEQ ID NO: 12,
(b) 0.8 mg/mL of poloxamer 188,
(c) 125 mM of arginine hydrochloride, and
(d) 50 mM of a histidine-acetate histidine buffer, wherein the pharmaceutical composition has a pH of 6.0.

In some embodiments, the pharmaceutical composition according to any one of the above, wherein the pharmaceutical composition is a liquid formulation. In some embodiments, the solvent of the liquid formulation is water.

The present disclosure further provides a lyophilised formulation comprising an anti-TSLP antibody, wherein the lyophilised formulation is obtained by lyophilising the pharmaceutical composition according to any one of the foregoing.

The present disclosure further provides a reconstituted solution, characterised in that the reconstituted solution is obtained by reconstituting the lyophilised formulation as described above.

The present disclosure further provides a reconstituted solution, which is a reconstituted dosage form of the lyophilised formulation as described above.

In some embodiments, the reconstituted solution according to any one of the above has the same components and amounts as those of the pharmaceutical composition described above.

In some embodiments, the reconstituted solution according to any one of the above comprises:

(a) 1 mg/mL to 250 mg/mL of an anti-TSLP antibody,
(b) 0.01 mg/mL to 2 mg/mL of a surfactant,
(c) 1 mM to 300 mM of an excipient, and
(d) 5 mM to 100 mM of a buffer, wherein the reconstituted solution has a pH from 5.0 to 7.0.

In some embodiments, the reconstituted solution according to any one of the above comprises:

(a) 1 mg/mL to 250 mg/mL of an anti-TSLP antibody,
(b) 0.01 mg/mL to 2 mg/mL of poloxamer,
(c) 1 mM to 300 mM of an excipient, and
(d) 5 mM to 100 mM of a buffer, wherein the reconstituted solution has a pH from 5.0 to 7.0.

In some embodiments, the reconstituted solution according to any one of the above comprises:

(a) 90 mg/mL to 200 mg/mL of an anti-TSLP antibody,
(b) 0.2 mg/mL to 2 mg/mL of poloxamer 188,
(c) 50 mM to 200 mM of arginine hydrochloride or proline, and
(d) 10 mM to 60 mM of a histidine-acetate histidine buffer, wherein the reconstituted solution has a pH from 5.0 to 6.5.

In some embodiments, the reconstituted solution according to any one of the above comprises:

(a) 90 mg/mL to 200 mg/mL of an anti-TSLP antibody,
(b) 0.2 mg/mL to 2 mg/mL of poloxamer 188,
(c) 50 mM to 200 mM of arginine hydrochloride or proline, and
(d) 10 mM to 50 mM of a histidine-acetate histidine buffer, wherein the reconstituted solution has a pH from 5.5 to 6.5.

In some embodiments, the reconstituted solution according to any one of the above comprises:

(a) 150 mg/mL to 200 mg/mL of an anti-TSLP antibody,
(b) 0.2 mg/mL to 1 mg/mL of poloxamer 188,
(c) 50 mM to 150 mM of arginine hydrochloride, and
(d) 35 mM to 55 mM of a histidine-acetate histidine buffer, wherein the reconstituted solution has a pH from 5.0 to 6.5.

In some embodiments, the reconstituted solution according to any one of the above comprises:

(a) 150 mg/mL to 200 mg/mL of an anti-TSLP antibody,
(b) 0.2 mg/mL to 1 mg/mL of poloxamer 188,
(c) 50 mM to 150 mM of arginine hydrochloride, and
(d) 32 mM to 48 mM of a histidine-acetate histidine buffer, wherein the reconstituted solution has a pH from 5.7 to 6.3.

In some embodiments, the reconstituted solution according to any one of the above comprises:

(a) 150 mg/mL to 200 mg/mL of an anti-TSLP antibody,
(b) 0.64 mg/mL to 0.96 mg/mL of poloxamer 188,
(c) 95 mM to 150 mM of arginine hydrochloride, and
(d) 35 mM to 55 mM of a histidine-acetate histidine buffer, wherein the reconstituted solution has a pH from 5.7 to 6.3.

In some embodiments, the reconstituted solution according to any one of the above comprises:

(a) 180 mg/mL to 200 mg/mL of an anti-TSLP antibody,
(b) 0.64 mg/mL to 0.96 mg/mL of poloxamer 188,
(c) 95 mM to 150 mM of arginine hydrochloride, and
(d) 32 mM to 48 mM of a histidine-acetate histidine buffer, wherein the reconstituted solution has a pH from 5.7 to 6.3.

In some embodiments, the reconstituted solution according to any one of the above comprises:

(a) about 182 mg/mL of an anti-TSLP antibody,
(b) about 0.8 mg/mL of poloxamer 188,

(c) about 110 mM of arginine hydrochloride, and
(d) about 40 mM of a histidine-acetate histidine buffer, wherein the pharmaceutical composition has a pH of about 6.0.

In some embodiments, the reconstituted solution according to any one of the above comprises:

(a) about 182 mg/mL of an anti-TSLP antibody,
(b) about 0.8 mg/mL of poloxamer 188,
(c) about 125 mM of arginine hydrochloride, and
(d) about 50 mM of a histidine-acetate histidine buffer, wherein the pharmaceutical composition has a pH of about 6.0.

In some embodiments, the reconstituted solution according to any one of the above comprises:

(a) 150 mg/mL to 200 mg/mL of an anti-TSLP antibody comprising a heavy chain of SEQ ID NO: 11 and a light chain of SEQ ID NO: 12,
(b) 0.2 mg/mL to 1 mg/mL of poloxamer 188,
(c) 50 mM to 150 mM of arginine hydrochloride, and
(d) 32 mM to 48 mM of a histidine-acetate histidine buffer, wherein the reconstituted solution has a pH from 5.7 to 6.3.

In some embodiments, the reconstituted solution according to any one of the above comprises:

(a) 180 mg/mL to 200 mg/mL of an anti-TSLP antibody,
(b) 0.2 mg/mL to 0.8 mg/mL of poloxamer 188,
(c) 50 mM to 125 mM of arginine hydrochloride, and
(d) 30 mM to 50 mM of a histidine-acetate histidine buffer, wherein the reconstituted solution has a pH from 5.7 to 6.3.

In some embodiments, the reconstituted solution according to any one of the above comprises:

(a) 180 mg/mL to 200 mg/mL of an anti-TSLP antibody comprising a heavy chain of SEQ ID NO: 11 and a light chain of SEQ ID NO: 12,
(b) 0.2 mg/mL to 0.8 mg/mL of poloxamer 188,
(c) 50 mM to 125 mM of arginine hydrochloride, and
(d) 30 mM to 50 mM of a histidine-acetate histidine buffer, wherein the reconstituted solution has a pH from 5.7 to 6.3.

In some embodiments, the reconstituted solution according to any one of the above comprises:

(a) 180 mg/mL to 200 mg/mL of an anti-TSLP antibody comprising a heavy chain of SEQ ID NO: 11 and a light chain of SEQ ID NO: 12,
(b) 0.64 mg/mL to 0.96 mg/mL of poloxamer 188,
(c) 95 mM to 150 mM of arginine hydrochloride, and
(d) 32 mM to 48 mM of a histidine-acetate histidine buffer, wherein the reconstituted solution has a pH from 5.7 to 6.3.

In some embodiments, the reconstituted solution according to any one of the above comprises:

(a) about 182 mg/mL of an anti-TSLP antibody comprising a heavy chain of SEQ ID NO: 11 and a light chain of SEQ ID NO: 12,
(b) about 0.8 mg/mL of poloxamer 188,
(c) about 110 mM of arginine hydrochloride, and
(d) about 40 mM histidine-acetate histidine buffer, wherein the reconstituted solution has a pH of about 6.0.

In some embodiments, the reconstituted solution according to any one of the above comprises:

(a) 182 mg/mL of an anti-TSLP antibody comprising a heavy chain of SEQ ID NO: 11 and a light chain of SEQ ID NO: 12,
(b) 0.8 mg/mL of poloxamer 188,
(c) 110 mM of arginine hydrochloride, and
(d) 40 mM histidine-acetate histidine buffer, wherein the reconstituted solution has a pH of 6.0.

In some embodiments, the reconstituted solution according to any one of the above comprises:

(a) 150 mg/mL to 200 mg/mL of an anti-TSLP antibody comprising a heavy chain of SEQ ID NO: 11 and a light chain of SEQ ID NO: 12,
(b) 0.64 mg/mL to 0.96 mg/mL of poloxamer 188,
(c) 100 mM to 150 mM of arginine hydrochloride, and
(d) 40 mM to 60 mM of a histidine-acetate histidine buffer, wherein the reconstituted solution has a pH from 5.7 to 6.3.

In some embodiments, the reconstituted solution according to any one of the above comprises:

(a) about 182 mg/mL of an anti-TSLP antibody comprising a heavy chain of SEQ ID NO: 11 and a light chain of SEQ ID NO: 12,
(b) about 0.8 mg/mL of poloxamer 188,
(c) about 125 mM of arginine hydrochloride, and
(d) about 50 mM histidine-acetate histidine buffer, wherein the reconstituted solution has a pH from 5.7 to 6.3.

In some embodiments, the reconstituted solution according to any one of the above comprises:

(a) 182 mg/mL of an anti-TSLP antibody comprising a heavy chain of SEQ ID NO: 11 and a light chain of SEQ ID NO: 12,
(b) 0.8 mg/mL of poloxamer 188,
(c) 125 mM of arginine hydrochloride, and
(d) 50 mM histidine-acetate histidine buffer, wherein the reconstituted solution has a pH of 6.0.

In some embodiments, the pharmaceutical composition, lyophilised formulation or reconstituted solution according to any one of the foregoing is a subcutaneous injection formulation, an intravenous injection formulation, an intraperitoneal injection formulation or an intramuscular injection formulation. In some embodiments, the pharmaceutical composition, lyophilised formulation or reconstituted solution according to any one of the foregoing is a subcutaneous injection formulation.
In some embodiments, the pharmaceutical composition, lyophilised formulation or reconstituted solution according to any one of the foregoing is suitable for intravenous injection, subcutaneous injection, intraperitoneal injection or intramuscular injection. In some embodiments, the pharmaceutical composition, lyophilised formulation or reconstituted solution according to any one of the foregoing is suitable for subcutaneous injection.
In some embodiments, the pharmaceutical composition, lyophilised formulation or reconstituted solution according to any one of the foregoing is used for preparing a medicament for intravenous injection, subcutaneous injection, intraperitoneal injection or intramuscular injection. In some embodiments, the pharmaceutical composition, lyophilised formulation or reconstituted solution according to any one of the foregoing is used for preparing a medicament for subcutaneous injection.
The present disclosure further provides a method for preparing the pharmaceutical composition according to any one of the foregoing, the method comprising a step of replacing the anti-TSLP antibody drug substance with a buffer.
The present disclosure further provides an article, which comprises a container containing the pharmaceutical composition according to any one of the foregoing, or the lyophilised formulation as described above, or the reconstituted solution according to any one of the foregoing.
The present disclosure further provides a method for treating a disease or condition, the method comprising administering to a subject a therapeutically effective amount of the pharmaceutical composition according to any one of the foregoing, or the lyophilised formulation as described above, or the reconstituted solution according to any one of the foregoing.
The present disclosure further provides use of the pharmaceutical composition according to any one of the foregoing, or the lyophilised formulation as described above, or the reconstituted solution according to any one of the foregoing in the manufacture of a medicament for treating a disease or condition.
The pharmaceutical composition according to any one of the foregoing, or the lyophilised formulation as described above, or the reconstituted solution according to any one of the foregoing described in the present disclosure can be used as a medicament for treating a disease or condition.
In some embodiments, the disease or condition is associated with TSLP.
In some embodiments, the disease or condition is selected from allergic diseases, cancers and immune diseases; the allergic diseases are selected from:asthma, idiopathic pulmonary fibrosis, atopic dermatitis, allergic conjunctivitis, allergic rhinitis, allergic sinusitis, urticaria, Netherton syndrome, eosinophilic oesophagitis, food allergy, allergic diarrhoea, eosinophilic gastroenteritis, allergic bronchopulmonary aspergillosis, allergic fungal sinusitis and chronic pruritus; the cancers are selected from:breast cancer, colon cancer, lung cancer, ovarian cancer and prostate cancer; the immune diseases are selected from:rheumatoid arthritis, chronic obstructive pulmonary disease, systemic

sclerosis, multiple sclerosis, keloid, ulcerative colitis, nasal polyposis, chronic eosinophilic pneumonia, eosinophilic bronchitis, coeliac disease, Churg-Strauss syndrome, eosinophilic myalgia syndrome, hypereosinophilic syndrome, eosinophilic granulomatosis with polyangiitis, inflammatory bowel disease, scleroderma, interstitial lung disease, fibrosis induced by chronic hepatitis B or C, radiation-induced fibrosis and wound healing-induced fibrosis.

## Specific Embodiments

### Glossary

To make it easier to understand the present disclosure, certain technical and scientific terms are specifically defined as follows. Unless otherwise explicitly defined herein, all other technical and scientific terms used herein have the meanings commonly understood by those of ordinary skill in the art to which the present disclosure belongs.

As used in the present disclosure, the singular forms "a", "an" and "the" include plural references, unless the context clearly indicates otherwise.

Unless the context clearly requires otherwise, in the specification and claims, the terms "comprise(s)", "have(has)", "include(s)" and the like are to be construed as meaning "including but not limited to", rather than in an exclusive or exhaustive sense.

The term "and/or" means including both "and" and "or". For example, the phrase "A, B and/or C" is intended to cover each of the following: A, B and C; A, B or C; A or C; A or B; B or C; A and C; A and B; B and C; A (alone); B (alone); and C (alone).

"Optional" or "optionally" means that the subsequently described event or circumstance may or may not occur, and that the description includes instances where the event or circumstance occurs and instances where it does not.

Those skilled in the art will understand that when reference is made to numerical ranges, endpoints or specific values, "about" may indicate within one or more standard deviations. Alternatively, "about" may indicate a range differing by up to 20% (i.e. ±20%). Since many numerical values used herein are determined through experimentation, those skilled in the art will understand that such determinations may vary and typically do vary between experiments. Due to such inherent variation, the values used herein should not be unduly limited. Accordingly, the term "about" is used to encompass variations from the specified value of ±20% or less, ±10% or less, ±5% or less, ±1% or less, ±0.5% or less, or ±0.1% or less.

Although the disclosure provides content ranges or content values, those skilled in the art will understand that such content ranges or values encompass acceptable error margins of the measured values.

The amino acid three-letter codes and single-letter codes used herein are as described in J. Biol. Chem., 243, p.3558 (1968).

The term "Thymic Stromal Lymphopoietin (TSLP)" refers to a type I four-α-helix bundle cytokine, which is an epithelial-cell-derived cytokine produced in response to pro-inflammatory stimuli, closely related to interleukin-7 (IL-7), and initiates allergic reactions by stimulating dendritic cells (DCs). It is an important factor in regulating human immune responses. The term "TSLP" includes variants, isoforms, homologues, orthologues and paralogues of TSLP.

The term "amino acid" refers to naturally occurring and synthetic amino acids, as well as amino acid analogues and amino acid mimetics that act in a manner similar to naturally occurring amino acids. Naturally occurring amino acids are those encoded by the genetic code, as well as those that are subsequently modified, such as hydroxyproline acid, γ-carboxyglutamic acid and O-phosphoserine. Amino acid analogues refer to compounds having the same basic chemical structure as naturally occurring amino acids (i.e. an α-carbon bound to hydrogen, a carboxyl group, an amino group and an R group), such as homoserine, norleucine, methionine sulfoxide and methionine methylsulfonium. Such analogues have modified R groups (e.g. norleucine) or modified peptide backbones, but retain the same basic chemical structure as naturally occurring amino acids. Amino acid mimetics refer to chemical compounds that have a structure different from the general chemical structure of amino acids, but act in a manner similar to naturally occurring amino acids.

The term "antibody" is used in the broadest sense and encompasses various antibody structures, including but not limited to monoclonal antibodies, polyclonal antibodies; monospecific antibodies, multispecific antibodies (e.g. bispecific antibodies); full-length antibodies and antigen-binding fragments (or antigen-binding portions), provided that they exhibit the desired antigen-binding activity. "Natural antibody" refers to an immunoglobulin molecule that occurs naturally. For example, a natural IgG antibody is an approximately 150,000-dalton heterotetrameric glycoprotein composed of two identical light chains and two identical heavy chains linked by disulphide bonds. From the N-terminus to the C-terminus, each heavy chain has a variable region (VH), also known as the variable heavy domain or heavy chain variable region, followed by a heavy chain constant region, wherein the IgG heavy chain constant region (CH) typically contains three constant domains (CH1, CH2 and CH3); similarly, from the N-terminus to the C-terminus, each light chain has a variable region (VL), also known as the variable light domain or light chain variable domain, followed by a constant light domain (light chain constant region, CL).

The term "variable region" or "variable domain" refers to the domain in the antibody heavy or light chain involved

in antigen binding. As used herein, the antibody heavy chain variable region (VH) and light chain variable region (VL) each comprise four conserved framework regions (FRs) and three complementarity-determining regions (CDRs). The term "complementarity-determining region" or "CDR" refers to regions within the variable domain that primarily contribute to antigen binding; "framework" or "FR" refers to the variable domain residues other than the CDR residues. VH contains three CDRs:HCDR1, HCDR2 and HCDR3; VL contains three CDRs:LCDR1, LCDR2 and LCDR3.Each VH or VL consists of three CDRs and four FRs arranged in the following order from the amino terminus (also known as the N-terminus) to the carboxyl terminus (also known as the C-terminus):FR1, CDR1, FR2, CDR2, FR3, CDR3, FR4. A single VH or VL may be sufficient to confer antigen-binding specificity.

Various well-known methods can be used to determine the amino acid sequence boundaries of the CDRs, for example:The "Kabat" numbering scheme (see Kabat et al. (1991),"Sequences of Proteins of Immunological Interest", 5th Ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001) and the ImMunoGenTics (IMGT) numbering scheme (Lefranc, M.P. et al., Dev Comp. Immunol., 27, 55-77 (2003); Front Immunol. 2018 Oct 16;9:2278); the correspondence among the various numbering systems is well known to those skilled in the art, for example, as shown in Table 1 below.

Table 1 Correspondence between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise specified, the variable region and CDR sequences in the embodiments of the present disclosure follow the "Kabat" numbering scheme.

The term "monoclonal antibody" refers to a substantially homogeneous population of antibodies, i.e. the amino acid sequences of the antibody molecules contained in the population are identical, except for possible minor naturally occurring mutations. In contrast, polyclonal antibody preparations typically comprise multiple different antibodies with different amino acid sequences in their variable domains, which usually specifically recognise different epitopes. "Monoclonal" refers to the characteristic of antibodies obtained from a substantially homogeneous antibody population and should not be interpreted as requiring antibodies to be produced by any particular method. In some embodiments, the antibodies provided by the present disclosure are monoclonal antibodies.

The antibodies of the present disclosure may be animal-derived antibodies (e.g. antibodies derived from mouse, avian, rabbit, camel or monkey), chimeric antibodies or humanised antibodies.

The term "chimeric antibody" refers to an antibody in which a portion of the heavy and/or light chain is derived from a particular source or species, and the remaining portion of the heavy and/or light chain is derived from a different source or species.

The term "humanised" antibody refers to an antibody that retains the reactivity of a non-human antibody while having reduced immunogenicity in humans. For example, this can be achieved by retaining non-human CDR regions and replacing the remaining portions of the antibody with their human counterparts (i.e. constant regions and portions of the framework regions of the variable domains).

The term "affinity" refers to the overall strength of non-covalent interactions between a single binding site of a molecule (e.g. an antibody) and its binding ligand (e.g. an antigen). Unless otherwise specified, as used herein, binding "affinity" refers to intrinsic binding affinity, reflecting a 1:1 interaction between the members of a binding pair (e.g. antibody and antigen). The affinity of a molecule X for its ligand Y is typically expressed by the dissociation constant (KD). Affinity can be measured by conventional methods known in the art.

As used herein, the term "kassoc" or "ka" refers to the association rate of a specific antibody-antigen interaction, and the term "kdis" or "kd" refers to the dissociation rate of a specific antibody-antigen interaction. The term "KD" refers to the dissociation constant, which is obtained from the ratio of kd to ka (i.e. kd/ka) and is expressed in molar concentration (M). Methods known in the art may be used to determine the KD value of an antibody. For example, affinity may be measured using biosensor systems such as systems measuring surface plasmon resonance, or by solution equilibrium titration (SET) to measure affinity in solution. In some embodiments, the KD value is determined by Biacore analysis.

The term "antigen" refers to a molecule or molecular moiety that can be bound by a selective binder such as an antigen-binding protein (e.g. an antibody), and that can also be used in animals to elicit the production of antibodies capable of binding the antigen. An antigen may have one or more epitopes that interact with different antigen-binding proteins (e.g. antibodies).

The term "epitope" refers to a region (area or region) on an antigen that specifically binds to an antibody or an antigen-binding fragment thereof. An epitope may be formed by a contiguous amino acid sequence (linear epitope) or may comprise non-contiguous amino acids (conformational epitope), for example, brought into spatial proximity by folding of the antigen (i.e. tertiary folding of a proteinaceous antigen). The difference between conformational epitopes and linear epitopes is that:in the presence of denaturing solvents, antibody binding to conformational epitopes is lost. An epitope comprises at least 3, at least 4, at least 5, at least 6, at least 7, or 8-10 amino acids in a unique spatial conformation. Screening antibodies that bind to a particular epitope (i.e. those that bind to the same epitope) can be carried out using routine methods in the art, including but not limited to alanine scanning, peptide blotting, peptide cleavage analysis, epitope excision, epitope extraction, chemical modification of antigens (see Prot. Sci. 9 (2000) 487-496) and cross-blocking.

The terms "capable of specifically binding", "specific binding" or "binding" mean that an antibody binds to an antigen or an epitope within the antigen with higher affinity compared with other antigens or epitopes. Typically, an antibody binds to an antigen or an epitope within the antigen with an equilibrium dissociation constant (KD) of about $1\times10$-7 M or less (e.g. less than 10 nM, 3 nM, 1 nM, 0.3 nM, 0.1 nM or less). In some embodiments, the KD for binding of an antibody to an antigen is 10% or less (e.g. 1%) of the KD for binding of the antibody to a non-specific antigen (e.g. BSA, casein). KD can be measured using known methods, for example, by surface plasmon resonance measurements using BIACORE®. However, antibodies that specifically bind to an antigen or an epitope within an antigen may exhibit cross-reactivity with other related antigens, for example, corresponding antigens from other species (homologues), such as humans or monkeys, for example cynomolgus monkey (Macaca fascicularis) (cynomolgus, cyno), chimpanzee (Pan troglodytes) (chimpanzee, chimp) or common marmoset (Callithrix jacchus) (marmoset).

The term sequence "identity" refers to the degree (percentage) to which amino acids/nucleic acids at equivalent positions are identical between two sequences when optimally aligned, with gaps introduced as necessary to achieve maximum sequence identity, and without counting conservative substitutions as part of sequence identity. To determine the percentage of sequence identity, alignment may be achieved by techniques known in the art, for example using publicly available computer software such as BLAST, BLAST-2, ALIGN, ALIGN-2 or Megalign (DNASTAR). Those skilled in the art can determine parameters suitable for measuring alignment, including any algorithms required to achieve maximum alignment over the full length of the sequences being compared.

"Surfactant" refers to a surface-active agent, preferably a non-ionic surfactant. The use of surfactants can reduce protein aggregation and/or the formation of particulates in the formulation. The amount of surfactant added is such that it can reduce protein aggregation in the formulation and minimise particulate formation.

"Poloxamer" is an $\alpha$-hydroxy-$\omega$-hydroxy poly(oxyethylene)$_a$-poly(oxypropylene)$_b$-poly(oxyethylene)$_a$ block copolymer. It is formed by reacting propylene oxide with propylene glycol to form polyoxypropylene glycol, followed by addition of ethylene oxide to form a block copolymer. Wherein a is the number of oxyethylene units and b is the number of oxypropylene units. Examples of poloxamers include poloxamer 188. Specifically, poloxamer 188 has 75-85 oxyethylene units (a) and 25-30 oxypropylene units (b) in the copolymer, an oxyethylene (EO) content of 79.9%-83.7%, and an average molecular weight of 7680-9510.

A "viscosity modifier" is a conventional pharmaceutical excipient added to adjust the viscosity of a formulation. Viscosity modifiers may be inorganic salts or amino acid salts; preferably, the inorganic salts are selected from sodium chloride, calcium chloride and magnesium chloride; preferably, the amino acid salts are selected from arginine hydrochloride, histidine hydrochloride, lysine hydrochloride, histidine acetate and the like.

"Buffer" refers to a buffer that resists pH changes through the action of its acid-base conjugate components. Examples of buffers for controlling pH within an appropriate range include acetates, succinates, gluconates, histidine, oxalates, lactates, phosphates, citrates (also known as citrate salts), tartrates, fumarates, glycylglycine and other organic acid buffers.

"Histidine buffer" is a buffer containing histidine. Examples of histidine buffers include histidine-acetate histidine, histidine-hydrochloride histidine, histidine-phosphate histidine and histidine-sulphate histidine buffers, preferably histidine-acetate histidine buffer. Histidine-acetate histidine buffer may be prepared from histidine and acetic acid, or from histidine and histidine acetate.

"Replacement" refers to the replacement of the solvent system dissolving antibody proteins, such as using the buffer system of a stable formulation to replace a high-salt or hyperosmotic solvent system containing antibody proteins through physical operations, thus allowing for the presence of antibody proteins in the stable formulation. Such physical operations include but are not limited to ultrafiltration, dialysis or centrifugation.

"Pharmaceutical composition" refers to a mixture comprising one or more antibodies described herein or physiologically/pharmaceutically acceptable salts or prodrugs thereof with other chemical components, as well as other

components such as physiologically/pharmaceutically acceptable carriers and excipients. The purpose of the pharmaceutical composition is to facilitate administration to a subject and to promote absorption of the active ingredient so as to exert bioactivity.

"Pharmaceutically acceptable carrier" or "pharmaceutically acceptable excipient" includes any material that, when combined with an active ingredient, allows the ingredient to retain biological activity and does not react with the immune system of the subject. Examples include but are not limited to any standard pharmaceutical carriers, such as phosphate buffered saline solutions, water, emulsions such as oil/water emulsions, and various types of wetting agents. In some embodiments, diluents for aerosol or parenteral administration are phosphate buffered saline (PBS) or physiological (0.9%) saline. Compositions containing such carriers are prepared by well-known conventional methods (see, for example, Remington's Pharmaceutical Sciences, 18th edition, A. Gennaro, editor, Mack Publishing Co., Easton, PA, 1990; and Remington, The Science and Practice of Pharmacy, 20th edition, Mack Publishing, 2000).

"Lyophilised formulation" refers to the formulation or pharmaceutical composition obtained after vacuum lyophilisation steps from a liquid or solution form of the pharmaceutical composition or liquid or solution formulation. Generally, freeze-drying comprises pre-freezing, primary drying and secondary drying. The purpose of pre-freezing is to freeze the product to obtain a crystalline solid; in some embodiments, the pre-freezing temperature is set at -45 °C and the pre-freezing rate is set at 1 °C/min. Primary drying, also referred to as main drying, is the main stage of freeze-drying, the purpose of which is to remove ice from the product while maintaining product shape and minimising damage to the product; improper selection of temperature and vacuum during primary drying may lead to collapse of the product; higher temperature and vacuum accelerate freeze-drying efficiency but also increase the risk of collapse. In some embodiments, the temperature of primary drying may be a conventional temperature in the art, for example -30 °C to 0 °C. Secondary drying, also referred to as desorption drying, is the main step for removing bound water from the product by applying ultimate vacuum (0.01 mbar) and increasing temperature (20 °C to 40 °C). Since most biological products are temperature-sensitive, the secondary drying temperature is often selected at the lower end of the temperature range, for example, 25 °C. The freeze-drying time depends on the freeze dryer, the dose of the lyophilised formulation and the container of the lyophilised drug. Adjustment of such time is well known to those skilled in the art.

The solution form of the pharmaceutical composition described in the present disclosure contains water as solvent unless otherwise specified.

In the present disclosure, "pharmaceutical composition" and "formulation" are not mutually exclusive. The pharmaceutical composition described in the present disclosure can achieve a stable effect: wherein the antibody substantially retains its physical stability and/or chemical stability and/or biological activity after storage; preferably, the pharmaceutical composition substantially retains its physical and chemical stability and its biological activity after storage. The storage period is generally selected based on the predetermined shelf life of the pharmaceutical composition. Currently, there are various analytical techniques available to determine protein stability, which can determine the stability after storage at selected temperatures over specified time periods.

Stable formulations include formulations in which no significant changes are observed under the following conditions: storage at refrigerated temperatures (2 °C to 8 °C, preferably 4 °C) for at least 1 month, at least 3 months, at least 6 months, preferably 1 year, and even more preferably up to 2 years. In addition, stable liquid formulations also includeliquid formulations that exhibit desired characteristics after storage at temperatures including 25 °C for periods including 1 month, 3 months or 6 months. Furthermore, stable liquid formulations also include those that exhibit desired characteristics after storage at 40 °C for periods including 4 weeks, 1 month, 3 months or 6 months. Typical examples of stability:as measured by SEC-HPLC, typically no more than about 10%, preferably no more than about 5%, of the antibody aggregates or degrades. By visual inspection, the formulation is a pale yellow to nearly colourless clear solution, or a colourless clear solution, or clear to slightly opalescent. The concentration, pH, weight and osmotic pressure of the formulation show changes of no more than about 10%, preferably no more than about 5%.

If, after visual inspection of colour and/or clarity, or as measured by UV light scattering, size-exclusion chromatography (SEC) and dynamic light scattering (DLS), the antibody does not show a significant increase in aggregation, precipitation and/or denaturation, then the antibody is considered to "retain its physical stability" in the pharmaceutical formulation. The changes in protein conformation can be evaluated by fluorescence spectroscopy (which determines tertiary structure of protein) and Fourier Transform Infrared (FTIR) spectroscopy (which determines secondary structure of protein).

If the antibody does not show significant chemical changes, then the antibody is considered to "retain its chemical stability" in the pharmaceutical formulation. The chemical stability can be assessed by detecting and quantifying the protein with its forms changed chemically. Degradation processes that commonly alter protein chemical structure include hydrolysis or truncation (evaluated by methods such as size-exclusion chromatography and CE-SDS), oxidation (evaluated by methods such as peptide mapping combined with mass spectrometry or MALDI/TOF/MS), deamidation (evaluated by methods such as ion exchange chromatography, capillary isoelectric focusing, peptide mapping, isoaspartate measurement) and isomerisation (evaluated by measuring isoaspartate content, peptide mapping, etc.).

If the biological activity of the antibody at a given time is within a predetermined range of the biological activity

exhibited at the time of preparation of the pharmaceutical formulation, then the antibody is considered to "retain its biological activity" in the pharmaceutical formulation.

The terms "administering", "giving" and "treating", when applied to an animal, human, study subject, cell, tissue, organ, or biological fluid, refer to the contact of an exogenous drug, therapeutic agent, diagnostic agent, or composition with the animal, human, subject, cell, tissue, organ, or biological fluid. The terms "administering", "delivery" and "treatment" may refer to, for example, therapeutic, pharmacokinetic, diagnostic, research and experimental methods. The treatment of cells includes the contact between reagents and cells, and between reagents and fluids, where the fluids are in contact with cells. The terms "administering", "giving" and "treating" also refer to the application of an agent, diagnostic, binding composition, or another type of ex vivo or in vitro manipulation, for example, of cells. "Treating", when applied to human, veterinary or research subjects, refers to therapeutic treatment, prophylaxis or preventive measures, research and diagnostic applications.

"Treatment" means administration of a therapeutic agent to a patient, either internally or externally, such as a pharmaceutical composition comprising any one described herein, wherein the patient has one or more disease symptoms and the therapeutic agent is known to have therapeutic effects on such symptoms. Typically, a therapeutic agent is administered in an amount effective to alleviate one or more disease symptoms in a patient or population under treatment, in order to induce regression of such symptoms or inhibit their progression to any clinically measurable extent. The amount of the therapeutic agent that effectively alleviates any specific disease symptom (also referred to as "therapeutically effective amount") may vary depending on several factors, such as the patient's disease state, age and body weight, as well as the drug's ability to produce the desired effect to the patient. Whether the disease symptom has alleviated can be evaluated by any clinical testing method commonly used by physicians or other healthcare professionals to evaluate the severity or progression of the symptom. Although the embodiments (such as therapeutic methods or products) in the present disclosure may be ineffective in alleviating each targeted disease symptom, yet any statistical testing method known in the field, such as Student's t-test, Chi-square test, Mann-Whitney U test, Kruskal-Wallis test (H-test), Jonckheere-Terpstra test and Wilcoxon test, can determine that they should alleviate the targeted disease symptoms in a statistically significant number of patients.

"Effective amount" includes an amount sufficient to ameliorate or prevent symptoms of a medical condition or disease. An effective amount also refers to an amount sufficient to allow for or facilitate diagnosis. An effective amount for a subject may vary depending on factors such as:the condition to be treated, the overall health of the subject, the route and dose of administration, and the severity of side effects. The effective amount may be the maximum dosage or dosing regimen that avoids significant side effects or toxic effects. Subjects of the present disclosure may be animals or human subjects.

The pharmaceutical compositions of the present disclosure may be administered by any suitable means, including parenteral, pulmonary and intranasal administration, and, if local treatment is required, intralesional administration. Parenteral infusion includes intramuscular, intravenous, intra-arterial, intraperitoneal or subcutaneous administration. Administration may be by any appropriate route, for example by injection, such as intravenous or subcutaneous injection. Various dosing schedules are contemplated herein, including but not limited to single administration or multiple administrations at multiple time points, bolus administration and pulse infusion. In some embodiments, the pharmaceutical compositions of the present disclosure are administered by subcutaneous injection.

The pharmaceutical compositions of the present disclosure will be formulated, administered and applied in accordance with good medical practice. Factors considered in this context include the specific condition being treated, the particular mammal being treated, the clinical condition of the individual patient, the cause of the condition, the site of delivery of the agent, the method of administration, the timing of administration, and other factors known to medical practitioners. Optionally, the pharmaceutical composition may also be formulated with one or more other agents for preventing or treating the condition. The effective amount of such other agents depends on the amount of antigen-binding molecule present in the pharmaceutical composition, the type of condition or treatment, and other factors. Such agents may be used at the same dose and route of administration as described herein, or at about 1 to 99% of the dose described herein, or at any dose, and by any route determined to be appropriate by empirical/clinical means.

Diseases related to TSLP in the present disclosure are not limited, as long as they are diseases associated with TSLP; for example, therapeutic responses induced by the antibodies of the present disclosure may be achieved by binding to human TSLP and thereby blocking the binding of TSLP to its receptor, or killing cells overexpressing TSLP; or inhibiting the growth of cells overexpressing TSLP.

Details of one or more embodiments of the present disclosure have been presented in the foregoing description. Although any methods and materials similar or equivalent to those described herein can be used in the practice or testing of the present disclosure, the preferred methods and materials are described below. Other features, objectives and advantages of the disclosure will be apparent from the specification and claims. In the specification and claims, unless the context clearly dictates otherwise, singular forms include the plural referents. Unless otherwise defined, all technical and scientific terms used herein have the same meaning as commonly understood by one of ordinary skill in the art to which this disclosure belongs. All patents and publications cited in this specification are incorporated herein by reference. The

following examples are provided to more fully illustrate the preferred embodiments of the present disclosure. These examples are not to be construed as limiting the scope of the disclosure in any way, which is defined by the claims.

## Examples

### Example 1: Anti-human TSLP monoclonal antibody

WO2020244544A1 has disclosed an anti-TSLP antibody hu179-33, and the amino acid sequences of the heavy chain CDRs and light chain CDRs of the hu179-33 antibody are shown in Table 2.

Table 2 CDR region sequences of the heavy chain and light chain of hu179-33 antibody

| Antibody | Heavy chain CDR | | Light chain CDR | |
|---|---|---|---|---|
| hu179-33 | HCDR1 | NYLIE SEQ ID NO: 1 | LCDR1 | KASQSVSSDVT SEQ ID NO: 4 |
| | HCDR2 | VIDPGVGDTNYNENFKG SEQ ID NO: 2 | LCDR2 | YVSEHYT SEQ ID NO: 5 |
| | HCDR3 | EDNTGTAFDY SEQ ID NO: 3 | LCDR3 | QQHHRFPLT SEQ ID NO: 6 |

The heavy chain variable region and light chain variable region, heavy chain constant region and light chain constant region, and heavy chain and light chain sequences of the hu179-33 antibody are as follows:

Heavy chain variable region of hu179-33 antibody (SEQ ID NO: 7):

EVQLVQSGAEVKKPGSSVKVSCKASGYTFSNYLIEWVRQAPGQGLEWIGV IDPGVGDTNYNENFKGRATLTADKSTSTAYIELSSLRSEDTAVYYCAREDNTGTA FDYWGQGTTVTVSS

Light chain variable region of hu179-33 antibody (SEQ ID NO: 8):

SIVMTQTPLSLSVTPGQPASISCKASQSVSSDVTWYLQKPGQSPQLLIYYVSEH YTGVPDRFSGSGYGTDFTLKISRVEAEDVGVYYCQQHHRFPLTFGQGTKLEIK

Heavy chain constant region of hu179-33 antibody (SEQ ID NO: 9):

ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKT HTCPPCPAPELLGGPSVFLFPPKPKDTLYITREPEVTCVVVDVSHEDPEVKFNWY VDGVEVHNAKTKPREEQYNSTYRVVSVLTVLHQDWLNGKEYKCKVSNKALPA PIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNHYTQ KSLSLSPGK

Light chain constant region of hu179-33 antibody (SEQ ID NO: 10):

RTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNRG EC

Heavy chain of hu179-33 antibody (SEQ ID NO: 11):

EVQLVQSGAEVKKPGSSVKVSCKASGYTFSNYLIEWVRQAPGQGLEWIGV IDPGVGDTNYNENFKGRATLTADKSTSTAYIELSSLRSEDTAVYYCAREDNTGTA FDYWGQGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVD KKVEPKSCDKTHTCPPCPAPELLGGPSVFLFPPKPKDTLYITREPEVTCVVVDVS HEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSV MHEALHNHYTQKSLSLSPGK

Light chain of hu179-33 antibody (SEQ ID NO: 12):

SIVMTQTPLSLSVTPGQPASISCKASQSVSSDVTWYLQKPGQSPQLLIYYVS EHYTGVPDRFSGSGYGTDFTLKISRVEAEDVGVYYCQQHHRFPLTFGQGTKLEI KRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC

**Example 2: Anti-TSLP antibody formulation**

The equipment, antibodies and methods used in formulation preparation and testing are as follows:

**SEC size-exclusion chromatography:**

An analytical method for separating solutes based on the relationship between the pore size of the gel and the coil size of macromolecular sample molecules.

SEC% (SEC monomer content percentage) = A_monomer / A_total × 100% (A_monomer is the peak area of the main monomer peak in the sample, and A_total is the sum of all peak areas). ΔSEC% = SEC% of the formulation before the stability study - SEC% of the formulation after the stability study.

SEC measuring instrument:Agilent HPLC 1260.
Column:Waters, XBridge® BEH200 Å SEC 3.5 μm 7.8 × 300 mm Column.

**NR-CE capillary gel electrophoresis:**

A type of electrophoresis where the gel is transferred into a capillary as a supporting medium, and also a method of separation based on the sample molecular weight under a certain voltage.

NR-CE% = A main peak / A total × 100% (A main peak is the peak area of the main peak in the sample, and A total is the sum of the areas of all peaks.) ΔNR-CE% = NR-CE% of the formulation before the stability study - NR-CE% of the formulation after the stability study.

CE measuring instrument:Beckman capillary electrophoresis system, model PA800 plus.

**IEC ion-exchange chromatography**:

A chromatographic method in which ion-exchange resins or chemically bonded ion exchangers are used as the stationary phase, and separation is achieved by differences in ion-exchange capacity or selectivity coefficients of the components to be separated.

IEC% = A neutral peak area / A total area × 100% (A total area is the sum of acidic peak, neutral peak and basic peak areas). ΔIEC% = IEC% of the formulation before the stability study - IEC% of the formulation after the stability study.

Instruments used for IEC determination:Agilent HPLC 1260.

**Osmolality determination:**

Osmotic pressure was determined using the freezing point method. Based on the directly proportional relationship between the freezing point depression and the molar concentration of the solution, the highly sensitive temperature-sensitive components were used to determine the freezing point of the solution and convert the electrical quantity into osmotic pressure.

Instruments used for osmolality determination: Rozer Loser, model OM815.

**Viscosity determination:**

The sample is loaded between two measuring plates. When a torque is applied to the upper plate, a rotational shear stress is generated in the material, and the resulting strain or strain rate is measured to calculate the viscosity.

Instruments used for viscosity determination:Anton Paar, model MCR 302.

**Protein**

The protein used in the following examples is the above-described anti-TSLP antibody hu179-33 (hereinafter referred to as "the protein").

Instruments used for determination of protein concentration:UV-visible spectrophotometer, model:NanoDrop OneC, optical path length 1 mm.

**Example 2-1: Surfactant screening-1**

Group 1-5 formulations containing 100 mg/mL protein, 70 mg/mL sucrose, and different types and concentrations of surfactants were prepared using 20 mM His-AA buffer (pH 6.0). The samples were subjected to forced degradation and long-term stability studies (40 °C for 1 month, 4 °C for 12 and 24 months), with appearance, SEC and IEC as evaluation indices, to investigate the effects of different types and concentrations of surfactants on protein stability. The results are shown in Table 3.

Table 3 Results of surfactant screening-1

| Group | Surfactant | Storage Conditions | Appearance | ΔSEC% | ΔIEC% |
|---|---|---|---|---|---|
| 1* | 0.8 mg/mL PS80 | 40°C M1 | Clear | 8.1 | 34.1 |
| | | 4°C M12 | Clear | 1.1 | 2.4 |
| | | 4°C M24 | Clear | 1.2 | 6.2 |
| 2 | 0.2 mg/mL PS80 | 40°C M1 | Clear | 4.1 | 17.7 |
| | | 4°C M12 | Clear | -0.2 | -1.7 |
| | | 4°C M24 | A small number of fine particles | 0.5 | -0.4 |
| 3 | 0.4 mg/mL PS80 | 40°C M1 | Clear | 5.5 | 24.6 |
| | | 4°C M12 | Clear | -0.1 | -0.8 |
| | | 4°C M24 | Clear | 0.7 | 1.1 |
| 4 | 0.2 mg/mL PF68 | 40°C M1 | Clear | 3.2 | 14.3 |
| | | 4°C M12 | Clear | -0.3 | -0.7 |
| | | 4°C M24 | Clear | 0.5 | 0.0 |

(continued)

| Group | Surfactant | Storage Conditions | Appearance | ΔSEC% | ΔIEC% |
|---|---|---|---|---|---|
| 5 | 0.8 mg/mL PF68 | 40°C M1 | Clear | 3.3 | 13.4 |
| | | 4°C M12 | Clear | -0.4 | -1.7 |
| | | 4°C M24 | Clear | 0.6 | -0.1 |
| Note: 20 mM His-AA buffer (pH 6.0) represents 20 mM histidine-acetate histidine (pH 6.0); PS80 represents polysorbate 80; PF68 represents poloxamer 188; 40 °C M1 represents storage at 40 °C for one month; 4 °C M12 represents storage at 4 °C for twelve months; 4 °C M24 represents storage at 4 °C for twenty-four months; the same applies below.<br>*: Group 1 is the first-generation formulation disclosed in WO2022116858A1. | | | | | |

The results show that:After storage at 40 °C for one month, based on SEC and IEC data, the stability ranking is: Groups 4-5 > Group 2 > Group 3 > Group 1; after storage at 4 °C for 12 or 24 months, the stability by SEC/IEC of Groups 2-5 is superior to that of Group 1, and there is no significant difference among Groups 2-5. In terms of appearance, Groups 1, 3, 4 and 5 are superior to Group 2.

In summary, appearance and purity data indicate that PF68 is superior to the other groups.

**Example 2-2: Surfactant screening-2**

Group 2-5 formulations containing 182 mg/mL protein and different surfactants were prepared using 50 mM His-AA and 125 mM arginine hydrochloride (pH 6.0) buffer. The samples were subjected to forced degradation studies (40 °C for one month), with appearance, SEC and IEC as evaluation indices, to investigate the effects of different surfactants on protein stability, while comparing with the first-generation formulation. The results are shown in Table 4.

Table 4 Results of surfactant screening-2

| Group | Surfactant | Storage Conditions | Appearance | ΔSEC% | ΔIEC% |
|---|---|---|---|---|---|
| 1* | 0.8 mg/mL PS80 | 40°C M1 | Clear | 8.1 | 34.1 |
| 2 | 0.8 mg/mL PS80 | 40°C M1 | Clear | 4.4 | 24.1 |
| 3 | 0.2 mg/mL PF68 | 40°C M1 | Clear | 3.5 | 14.7 |
| 4 | 0.8 mg/mL PF68 | 40°C M1 | Clear | 3.3 | 14.6 |
| 5 | 2 mg/mL PF68 | 40°C M1 | Clear | 3.5 | 14.4 |
| *: Group 1 is the first-generation formulation disclosed in WO2022116858A1. | | | | | |

The results show that: After storage at 40 °C for one month, the PF68 groups at 0.2-2 mg/mL show no significant differences in appearance and purity, and are all significantly superior to the PS80 group and the first-generation formulation.

**Example 2-3: Excipient screening-1**

Group 1-6 formulations containing 150 mg/mL protein and different types and concentrations of excipients were prepared using 20 mM His-AA buffer (pH 6.0), with viscosity as the evaluation index; the specific formulations are shown in Table 5:

Table 5 Results of excipient screening-1

| Group | Buffer | Excipient | Protein Concentration | Viscosity (cP) |
|---|---|---|---|---|
| 1 | 20 mM His-AA (pH 6.0) | N/A | 150 mg/mL | 16.70 |
| 2 | | 50 mM arginine hydrochloride | | 10.47 |
| 3 | | 125 mM arginine hydrochloride | | 7.23 |
| 4 | | 50 mM proline | | 17.63 |

(continued)

| Group | Buffer | Excipient | Protein Concentration | Viscosity (cP) |
|---|---|---|---|---|
| 5 | | 100 mM proline | | 17.38 |
| 6 | | 200 mM proline | | 15.29 |

The results show that the arginine hydrochloride groups exhibit superior viscosity compared with the other groups; therefore, arginine hydrochloride is selected as the viscosity-reducing agent.

**Example 2-4: Excipient screening-2**

Group 2-4 formulations containing 182 mg/mL protein and 0.8 mg/mL PF68 were prepared using 50 mM His-AA and different concentrations of arginine hydrochloride (pH 6.0) buffer. The samples were subjected to forced degradation studies (40 °C for one month), with appearance, SEC, NR-CE and IEC as evaluation indices, to investigate the effects of different concentrations of arginine hydrochloride on protein stability, while comparing with the first-generation formulation. The results are shown in Table 6.

Table 6 Results of excipient screening-2

| Group | Arginine hydrochloride content | Storage Conditions | Appearance | ΔSEC% | ΔNR-CE% | ΔIEC% |
|---|---|---|---|---|---|---|
| 1* | N/A | 40°C M1 | Clear | 8.1 | 7.3 | 34.1 |
| 2 | 50mM | 40°C M1 | Clear | 3.4 | 3.4 | 14.7 |
| 3 | 125mM | 40°C M1 | Clear | 3.3 | 3.6 | 14.6 |
| 4 | 150mM | 40°C M1 | Clear | 3.5 | 3.7 | 13.6 |
| *: Group 1 is the first-generation formulation disclosed in WO2022116858A1. | | | | | | |

The results show that:After storage at 40 °C for one month, the 50-150 mM arginine hydrochloride groups show no significant differences in appearance and purity, and are all significantly superior to the first-generation formulation.

**Example 2-5: Protein concentration screening-1**

Group 1-6 formulations containing 125 mM arginine hydrochloride and different protein concentrations were prepared using 20 mM His-AA buffer (pH 6.0), with viscosity as the evaluation index; the results are shown in Table 7:

Table 7 Results of protein concentration screening-1

| Group | Buffer | Excipient | Protein Concentration | Viscosity (cP) |
|---|---|---|---|---|
| 1 | 20mM His-AA (pH 6.0) | N/A | 150 mg/mL | 16.70 |
| 2 | | 125 mM arginine hydrochloride | | 7.23 |
| 3 | | N/A | 180 mg/mL | 39.46 |
| 4 | | 125 mM arginine hydrochloride | | 14.24 |
| 5 | | N/A | 200 mg/mL | 78.55 |
| 6 | | 125 mM arginine hydrochloride | | 20.69 |

The results show that 125 mM arginine hydrochloride has a good viscosity-reducing effect, and when the protein concentration does not exceed 200 mg/mL, the formulation viscosity does not exceed 21 cP.

**Example 2-6: Protein concentration screening-2**

Group 2-4 formulations containing different protein concentrations and 0.8 mg/mL PF68 were prepared using 50 mM His-AA and 125 mM arginine hydrochloride (pH 6.0) buffer. The samples were subjected to forced degradation studies (40 °C for one month), with appearance, SEC, NR-CE and IEC as evaluation indices, to investigate the stability of

formulations with different protein concentrations, while comparing with the first-generation formulation. The results are shown in Table 8.

Table 8 Results of protein concentration screening-2

| Group | Protein Concentration | Storage Conditions | Appearance | ΔSEC% | ΔNR-CE% | ΔIEC% |
|---|---|---|---|---|---|---|
| 1* | 100 mg/mL | 40°C M1 | Clear | 8.1 | 7.3 | 34.1 |
| 2 | 90 mg/mL | 40°C M1 | Clear | 2.8 | 3.7 | 13.8 |
| 3 | 182mg/mL | 40°C M1 | Clear | 3.3 | 3.6 | 14.6 |
| 4 | 200 mg/mL | 40°C M1 | Clear | 3.7 | 4.0 | 14.6 |
| *: Group 1 is the first-generation formulation disclosed in WO2022116858A1. | | | | | | |

The results show that:After storage at 40 °C for one month, the 90-200 mg/mL groups show no significant differences in appearance and purity, and are all significantly superior to the first-generation formulation.

**Example 2-7: Buffer system ionic strength screening-1**

Group 2-4 formulations containing 182 mg/mL protein and 0.8 mg/mL PF68 were prepared using His-AA buffers of different ionic strengths and 125 mM arginine hydrochloride (pH 6.0). The samples were subjected to forced degradation studies (40 °C for one month), with SEC, NR-CE and IEC as evaluation indices, to investigate the effects of buffer system ionic strength on protein stability, while comparing with the first-generation formulation. The results are shown in Table 9.

Table 9 Results of buffer system ionic strength screening-1

| Group | Buffer system ionic strength | Storage Conditions | ΔSEC% | ΔNR-CE% | ΔIEC% |
|---|---|---|---|---|---|
| 1* | 20mM His-AA | 40°C M1 | 8.1 | 7.3 | 34.1 |
| 2 | 10mM His-AA | 40°C M1 | 3.3 | 2.9 | 12.2 |
| 3 | 50mM His-AA | 40°C M1 | 3.3 | 3.6 | 14.6 |
| 4 | 60mM His-AA | 40°C M1 | 3.3 | 3.5 | 14.1 |
| *: Group 1 is the first-generation formulation disclosed in WO2022116858A1. | | | | | |

The results show that:After storage at 40 °C for one month, the 10-60 mM His-AA groups show no significant differences in purity results, and are all significantly superior to the first-generation formulation.

**Example 2-8: Buffer system ionic strength screening-2**

As the protein is a macromolecule and contributes relatively little to osmolality in the formulation, the factors affecting osmolality are mainly the buffer system and excipients. In Example 2-3, excipient screening determined the use of arginine hydrochloride as the viscosity-reducing agent, and Example 2-7 showed that when the ionic strength of His-AA is 10-60 mM, there is no significant difference in formulation purity; therefore, in this example, Group 1-2 formulations containing 125 mM arginine hydrochloride were prepared using His-AA buffers of different ionic strengths (pH 6.0), with osmolality as the evaluation index, to further screen the buffer system ionic strength. The specific formulations are shown in Table 10:

Table 10 Results of buffer system ionic strength screening-2

| Group | Buffer system | Excipient | Osmolality (mOsm) |
|---|---|---|---|
| 1 | 30mM His-AA 6.0 | 125 mM arginine hydrochloride | 260 |
| 2 | 50mM His-AA 6.0 | | 287 |

The results show that 30-50 mM His-AA buffer (pH 6.0) provides more favourable osmolality and meets isotonic requirements, among which 50 mM His-AA buffer (pH 6.0) is more favourable.

**Example 2-9: pH screening**

Group 2-4 formulations containing 182 mg/mL protein and 0.8 mg/mL PF68 were prepared using 50 mM His-AA and 125 mM arginine hydrochloride buffers of different pH values. The samples were subjected to forced degradation studies (40 °C for one month), with SEC, NR-CE and IEC as evaluation indices, to investigate the effects of different pH values on protein stability, while comparing with the first-generation formulation. The results are shown in Table 11.

Table 11 Results of pH screening

| Group | pH | Storage Conditions | △SEC% | △NR-CE% | △IEC% |
|---|---|---|---|---|---|
| 1* | 6.0 | 40°C M1 | 8.1 | 7.3 | 34.1 |
| 2 | 5.0 | 40°C M1 | 4.1 | 3.7 | 19.0 |
| 3 | 6.0 | 40°C M1 | 3.3 | 3.6 | 14.6 |
| 4 | 6.5 | 40°C M1 | 4.0 | 4.0 | 14.3 |
| *: Group 1 is the first-generation formulation disclosed in WO2022116858A1. | | | | | |

The results show that:After stored at 40 °C for one month, the formulations in pH 5.0-6.5 groups have all purity results within an acceptable range, and are all significantly superior to the first-generation formulation.

**Example 2-10: Formulation stability comparison**

Group 2-3 formulations containing 0.8 mg/mL PF68 and different protein concentrations were prepared using 50 mM His-AA and 125 mM arginine hydrochloride buffer (pH 6.0). The samples were subjected to forced degradation (stored at 40 °C for one month), with SEC, NR-CE and IEC as evaluation indices, to investigate the stability of the Group 2-3 formulations, while compared with the first-generation formulation. The results are shown in Table 12.

Table 12 Results of formulation stability comparison

| Group | Protein Concentration | Surfactant | Storage Conditions | ΔSEC% | ΔNR-CE% | ΔIEC% |
|---|---|---|---|---|---|---|
| 1* | 100 mg/mL | 0.8 mg/mL PS80 | 40°C M1 | 8.1 | 7.3 | 34.1 |
| 2 | 180mg/mL | 0.8 mg/mL PF68 | 40°C M1 | 3.2 | 3.6 | 11.9 |
| 3 | 200mg/mL | 0.8 mg/mL PF68 | 40°C M1 | 3.5 | 3.9 | 11.8 |
| *: Group 1 is the first-generation formulation disclosed in WO2022116858A1. | | | | | | |

SEC, NR-CE and IEC data show that after being stored at 40 °C for one month, the Group 2-3 formulations have the stability significantly superior to that of the first-generation formulation.

**Claims**

1. A pharmaceutical composition comprising an anti-TSLP antibody, a surfactant and excipients, wherein the surfactant is poloxamer, and the anti-TSLP antibody comprises a heavy chain variable region and a light chain variable region, wherein the heavy chain variable region comprises HCDR1 as shown in SEQ ID NO: 1, HCDR2 as shown in SEQ ID NO: 2, and HCDR3 as shown in SEQ ID NO: 3; and the light chain variable region comprises LCDR1 as shown in SEQ ID NO: 4, LCDR2 as shown in SEQ ID NO: 5, and LCDR3 as shown in SEQ ID NO: 6;

   Preferably, the heavy chain variable region comprises the amino acid sequence as shown in SEQ ID NO: 7, and the light chain variable region comprises the amino acid sequence as shown in SEQ ID NO: 8;
   More preferably, the anti-TSLP antibody comprises a heavy chain as shown in SEQ ID NO: 11, and a light chain as shown in SEQ ID NO: 12.

2. The pharmaceutical composition according to claim 1, wherein the surfactant is poloxamer 188.

3. The pharmaceutical composition according to claim 1 or 2, wherein the excipients are selected from arginine

hydrochloride, proline, histidine hydrochloride, lysine hydrochloride, calcium chloride, magnesium chloride and sodium chloride;

Preferably, the excipient is arginine hydrochloride or proline;
More preferably, the excipient is arginine hydrochloride.

**4.** The pharmaceutical composition according to any one of claims 1 to 3, wherein the anti-TSLP antibody has a concentration from 1 mg/mL to 250 mg/mL;

Preferably, the anti-TSLP antibody has a concentration from 90 mg/mL to 200 mg/mL;
More preferably, the anti-TSLP antibody has a concentration from 150 mg/mL to 200 mg/mL;
Further preferably, the anti-TSLP antibody has a concentration from 180 mg/mL to 200 mg/mL;
Most preferably, the anti-TSLP antibody has a concentration of about 182 mg/mL.

**5.** The pharmaceutical composition according to any one of claims 1 to 4, wherein the surfactant has a concentration from 0.01 mg/mL to 2 mg/mL;

Preferably, the surfactant has a concentration from 0.2 mg/mL to 2 mg/mL;
More preferably, the surfactant has a concentration from 0.2 mg/mL to 1 mg/mL;
Further preferably, the surfactant has a concentration from 0.64 mg/mL to 0.96 mg/mL;
Most preferably, the concentration is about 0.8 mg/mL.

**6.** The pharmaceutical composition according to any one of claims 1 to 5, wherein the excipient has a concentration from 1 mM to 300 mM;

Preferably, the excipient has a concentration from 50 mM to 200 mM;
More preferably, the excipient has a concentration from 50 mM to 150 mM;
Further preferably, the excipient has a concentration from 95 mM to 150 mM;
Most preferably, the excipient has a concentration of about 110 mM or the excipient has a concentration of about 125 mM.

**7.** The pharmaceutical composition according to any one of claims 1 to 6, further comprising a buffer, wherein the buffer is a histidinate buffer;
Preferably, the buffer is a histidine-acetate histidine buffer.

**8.** The pharmaceutical composition according to claim 7, wherein the buffer has a concentration from 5 mM to 100 mM;

Preferably, the buffer has a concentration from 10 mM to 60 mM; or
the buffer has a concentration from 10 mM to 50 mM;
Further preferably, the buffer has a concentration from 35 mM to 55 mM; or
the buffer has a concentration from 32 mM to 48 mM;
More preferably, the buffer has a concentration of about 50 mM; or
the buffer has a concentration of about 40 mM.

**9.** The pharmaceutical composition according to any one of claims 1 to 8, wherein the pH of the pharmaceutical composition is from 5.0 to 7.0;

Preferably, the pH of the pharmaceutical composition is from 5.0 to 6.5;
More preferably, the pH of the pharmaceutical composition is from 5.5 to 6.5;
Further preferably, the pH of the pharmaceutical composition is from 5.7 to 6.3;
Most preferably, the pH of the pharmaceutical composition is about 6.0.

**10.** The pharmaceutical composition according to any one of claims 1 to 9, comprising:

(a) 1 mg/mL to 250 mg/mL of an anti-TSLP antibody,
(b) 0.01 mg/mL to 2 mg/mL of poloxamer,
(c) 1 mM to 300 mM of an excipient, and
(d) 5 mM to 100 mM of a buffer, wherein the pharmaceutical composition has a pH from 5.0 to 7.0;

Preferably, the pharmaceutical composition comprises:

(a) 90 mg/mL to 200 mg/mL of an anti-TSLP antibody,
(b) 0.2 mg/mL to 2 mg/mL of poloxamer 188,
(c) 50 mM to 200 mM of arginine hydrochloride or proline, and
(d) 10 mM to 60 mM of a histidine-acetate histidine buffer, wherein the pharmaceutical composition has a pH from 5.0 to 6.5;

or

(a) 90 mg/mL to 200 mg/mL of an anti-TSLP antibody,
(b) 0.2 mg/mL to 2 mg/mL of poloxamer 188,
(c) 50 mM to 200 mM of arginine hydrochloride or proline, and
(d) 10 mM to 50 mM of a histidine-acetate histidine buffer, wherein the pharmaceutical composition has a pH from 5.5 to 6.5;

More preferably, the pharmaceutical composition comprises:

(a) 150 mg/mL to 200 mg/mL of an anti-TSLP antibody,
(b) 0.2 mg/mL to 1 mg/mL of poloxamer 188,
(c) 50 mM to 150 mM of arginine hydrochloride, and
(d) 35 mM to 55 mM of a histidine-acetate histidine buffer, wherein the pharmaceutical composition has a pH from 5.0 to 6.5;

or

(a) 150 mg/mL to 200 mg/mL of an anti-TSLP antibody,
(b) 0.2 mg/mL to 1 mg/mL of poloxamer 188,
(c) 50 mM to 150 mM of arginine hydrochloride, and
(d) 32 mM to 48 mM of a histidine-acetate histidine buffer, wherein the pharmaceutical composition has a pH from 5.7 to 6.3;

Further preferably, the pharmaceutical composition comprises:

(a) 150 mg/mL to 200 mg/mL of an anti-TSLP antibody,
(b) 0.64 mg/mL to 0.96 mg/mL of poloxamer 188,
(c) 95 mM to 150 mM of arginine hydrochloride, and
(d) 35 mM to 55 mM of a histidine-acetate histidine buffer, wherein the pharmaceutical composition has a pH from 5.7 to 6.3;

or

(a) 180 mg/mL to 200 mg/mL of an anti-TSLP antibody,
(b) 0.64 mg/mL to 0.96 mg/mL of poloxamer 188,
(c) 95 mM to 150 mM of arginine hydrochloride, and
(d) 32 mM to 48 mM of a histidine-acetate histidine buffer, wherein the pharmaceutical composition has a pH from 5.7 to 6.3;

Most preferably, the pharmaceutical composition comprises:

(a) about 182 mg/mL of an anti-TSLP antibody,
(b) about 0.8 mg/mL of poloxamer 188,
(c) about 110 mM of arginine hydrochloride, and
(d) about 40 mM of a histidine-acetate histidine buffer, wherein the pharmaceutical composition has a pH of about 6.0;

or

(a) about 182 mg/mL of an anti-TSLP antibody,
(b) about 0.8 mg/mL of poloxamer 188,
(c) about 125 mM of arginine hydrochloride, and
(d) about 50 mM of a histidine-acetate histidine buffer, wherein the pharmaceutical composition has a pH of about 6.0.

**11.** The pharmaceutical composition according to any one of claims 1 to 10, which is a subcutaneous injection formulation, an intravenous injection formulation, an intraperitoneal injection formulation or an intramuscular injection formulation; preferably a subcutaneous injection formulation.

**12.** A lyophilised formulation comprising an anti-TSLP antibody, wherein the lyophilised formulation is obtained by freeze-drying the pharmaceutical composition according to any one of claims 1 to 11.

**13.** A method for preparing the pharmaceutical composition according to any one of claims 1 to 11, wherein the method comprises a step of replacing the anti-TSLP antibody drug substance with a buffer.

**14.** An article comprising a container, wherein the container contains the pharmaceutical composition according to any one of claims 1 to 11 or the lyophilised formulation according to claim 12.

**15.** A method for treating a disease or condition, wherein the method comprises administering to a subject a therapeutically effective amount of the pharmaceutical composition according to any one of claims 1 to 11, or the lyophilised formulation according to claim 12, wherein the disease or condition is selected from allergic diseases, cancers and immune diseases;

The allergic diseases are selected from:asthma, idiopathic pulmonary fibrosis, atopic dermatitis, allergic conjunctivitis, allergic rhinitis, allergic sinusitis, urticaria, Netherton syndrome, eosinophilic oesophagitis, food allergy, allergic diarrhoea, eosinophilic gastroenteritis, allergic bronchopulmonary aspergillosis, allergic fungal sinusitis and chronic pruritus;
The cancers are selected from:breast cancer, colon cancer, lung cancer, ovarian cancer and prostate cancer;
The immune diseases are selected from:rheumatoid arthritis, chronic obstructive pulmonary disease, systemic sclerosis, multiple sclerosis, keloid, ulcerative colitis, nasal polyposis, chronic eosinophilic pneumonia, eosinophilic bronchitis, coeliac disease, Churg-Strauss syndrome, eosinophilic myalgia syndrome, hypereosinophilic syndrome, eosinophilic granulomatosis with polyangiitis, inflammatory bowel disease, scleroderma, interstitial lung disease, fibrosis induced by chronic hepatitis B or C, radiation-induced fibrosis and wound healing-induced fibrosis.

**INTERNATIONAL SEARCH REPORT**

International application No.
**PCT/CN2024/125181**

**A. CLASSIFICATION OF SUBJECT MATTER**

C12N15/13(2006.01)i; A61K39/395(2006.01)i; A61K9/19(2006.01)i; A61K47/18(2017.01)i; A61K47/10(2017.01)i; A61P35/00(2006.01)i; A61P37/00(2006.01)i

According to International Patent Classification (IPC) or to both national classification and IPC

**B. FIELDS SEARCHED**

Minimum documentation searched (classification system followed by classification symbols)

IPC: C12N A61K A61P

Documentation searched other than minimum documentation to the extent that such documents are included in the fields searched

Electronic data base consulted during the international search (name of data base and, where practicable, search terms used)

CNABS, DWPI, SIPOABS, CNTXT, EPTXT, USTXT, WOTXT, PUBMED, CNKI, NCBI, EBI, STN, 中国专利生物序列检索系统, China Patent Biological Sequence Search System, 百度学术, Baidu Scholar: 申请人, Applicants, 发明人, Inventors, TSLP, 胸腺基质淋巴细胞生成素, 抗体, antibody, 组合物, composition, 泊洛沙姆, pluronic F68, poloxamer, 组氨酸, histidine, 缓冲液, buffer, SEQ ID NOs: 1-12

**C. DOCUMENTS CONSIDERED TO BE RELEVANT**

| Category* | Citation of document, with indication, where appropriate, of the relevant passages | Relevant to claim No. |
|---|---|---|
| Y | WO 2022116858 A1 (JIANGSU HENGRUI MEDICINE CO., LTD. et al.) 09 June 2022 (2022-06-09)<br>abstract, and claims 1-19 | 1-15 |
| Y | CN 116172947 A (JIANGSU HENGRUI MEDICINE CO., LTD. et al.) 30 May 2023 (2023-05-30)<br>claim 8 | 1-15 |
| Y | US 2014227250 A1 (MERCK SHARP & DOHME CORP.) 14 August 2014 (2014-08-14)<br>description, paragraphs 5-6 | 1-15 |
| Y | WO 2022184074 A1 (CHIA TAI TIANQING PHARMACEUTICAL GROUP CO., LTD.) 09 September 2022 (2022-09-09)<br>claims 1-15, and description, page 3, paragraph 1 | 1-15 |
| Y | CN 115279404 A (AMGEN INC.) 01 November 2022 (2022-11-01)<br>claims 1-122, and description, paragraph 113 | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 January 2025** | **17 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/125181**

**C. DOCUMENTS CONSIDERED TO BE RELEVANT**

| Category* | Citation of document, with indication, where appropriate, of the relevant passages | Relevant to claim No. |
|---|---|---|
| A | WO 2021139758 A1 (JIANGSU HENGRUI MEDICINE CO., LTD. et al.) 15 July 2021 (2021-07-15) claims 1-48 | 1-15 |
| A | WO 2022237882 A1 (JIANGSU HENGRUI MEDICINE CO., LTD. et al.) 17 November 2022 (2022-11-17) claims 1-22 | 1-15 |
| A | WO 2020244544 A1 (JIANGSU HENGRUI MEDICINE CO., LTD. et al.) 10 December 2020 (2020-12-10) claims 1-17 | 1-15 |

Form PCT/ISA/210 (second sheet) (July 2022)

**INTERNATIONAL SEARCH REPORT**

International application No.
**PCT/CN2024/125181**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/125181**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

Claim 15 relates to the prevention or treatment of diseases, which falls within subject matter for which no search is required by the International Searching Authority as defined in PCT Rule 39.1(iv). The international search is provided on the basis of the subject matter of the relevant claim is reasonably expected to be the use in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/125181**

| Patent document cited in search report | | | Publication date (day/month/year) | Patent family member(s) | | | Publication date (day/month/year) |
|---|---|---|---|---|---|---|---|
| WO | 2022116858 | A1 | 09 June 2022 | EP | 4257604 | A1 | 11 October 2023 |
| | | | | EP | 4257604 | A4 | 13 November 2024 |
| | | | | MX | 2023006596 | A | 19 June 2023 |
| | | | | JP | 2024500308 | A | 09 January 2024 |
| | | | | US | 2024016931 | A1 | 18 January 2024 |
| | | | | TW | 202228776 | A | 01 August 2022 |
| | | | | KR | 20230116857 | A | 04 August 2023 |
| | | | | CA | 3200767 | A1 | 09 June 2022 |
| CN | 116172947 | A | 30 May 2023 | None | | | |
| US | 2014227250 | A1 | 14 August 2014 | TW | 201420601 | A | 01 June 2014 |
| | | | | AR | 092216 | A1 | 08 April 2015 |
| | | | | WO | 2014031718 | A1 | 27 February 2014 |
| WO | 2022184074 | A1 | 09 September 2022 | None | | | |
| CN | 115279404 | A | 01 November 2022 | US | 2023078678 | A1 | 16 March 2023 |
| | | | | CO | 2022012868 | A2 | 09 December 2022 |
| | | | | MX | 2022010012 | A | 07 September 2022 |
| | | | | JOP | 20220183 | A1 | 30 January 2023 |
| | | | | UY | 39083 | A | 31 August 2021 |
| | | | | CL | 2022002193 | A1 | 24 March 2023 |
| | | | | AU | 2021219839 | A1 | 25 August 2022 |
| | | | | CR | 20220457 | A | 09 January 2023 |
| | | | | CA | 3166964 | A1 | 19 August 2021 |
| | | | | JP | 2023513312 | A | 30 March 2023 |
| | | | | PE | 20230112 | A1 | 27 January 2023 |
| | | | | EP | 4103235 | A1 | 21 December 2022 |
| | | | | BR | 112022016010 | A2 | 20 December 2022 |
| | | | | WO | 2021163504 | A1 | 19 August 2021 |
| | | | | IL | 295042 | A | 01 September 2022 |
| | | | | KR | 20220140772 | A | 18 October 2022 |
| WO | 2021139758 | A1 | 15 July 2021 | TW | 202128770 | A | 01 August 2021 |
| | | | | US | 2023120270 | A1 | 20 April 2023 |
| | | | | AU | 2021205561 | A1 | 11 August 2022 |
| | | | | JP | 2023509212 | A | 07 March 2023 |
| | | | | CA | 3164979 | A1 | 15 July 2021 |
| | | | | KR | 20220123072 | A | 05 September 2022 |
| | | | | EP | 4089111 | A1 | 16 November 2022 |
| | | | | EP | 4089111 | A4 | 04 October 2023 |
| WO | 2022237882 | A1 | 17 November 2022 | EP | 4339213 | A1 | 20 March 2024 |
| | | | | MX | 2023013307 | A | 04 December 2023 |
| | | | | KR | 20240007196 | A | 16 January 2024 |
| | | | | AU | 2022273737 | A1 | 16 November 2023 |
| | | | | US | 2024392020 | A1 | 28 November 2024 |
| | | | | CA | 3219388 | A1 | 17 November 2022 |
| | | | | TW | 202311295 | A | 16 March 2023 |
| | | | | JP | 2024517907 | A | 23 April 2024 |
| WO | 2020244544 | A1 | 10 December 2020 | JP | 2022535808 | A | 10 August 2022 |
| | | | | EP | 3981788 | A1 | 13 April 2022 |
| | | | | EP | 3981788 | A4 | 22 March 2023 |
| | | | | AU | 2020286889 | A1 | 20 January 2022 |

Form PCT/ISA/210 (patent family annex) (July 2022)

**INTERNATIONAL SEARCH REPORT**
**Information on patent family members**

International application No.
**PCT/CN2024/125181**

| Patent document cited in search report | Publication date (day/month/year) | Patent family member(s) | | | Publication date (day/month/year) |
|---|---|---|---|---|---|
| | | TW | 202110882 | A | 16 March 2021 |
| | | ZA | 202110236 | B | 31 August 2022 |
| | | KR | 20220016897 | A | 10 February 2022 |
| | | BR | 112021024112 | A2 | 22 March 2022 |
| | | MX | 2021014804 | A | 18 January 2022 |
| | | CA | 3142545 | A1 | 10 December 2020 |
| | | US | 2024254215 | A1 | 01 August 2024 |
| | | US | 2022340654 | A1 | 27 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311342148
- CN 202410995421
- WO 2020244544 A1
- WO 2022116858 A1

### Non-patent literature cited in the description

- **JOHANNA WEBER et al.** *International journal of pharmaceutics: X*, 2023, vol. 27 (6), 100202
- *J. Biol. Chem.*, 1968, vol. 243, 3558
- **KABAT et al.** Sequences of Proteins of Immunological Interest. National Institutes of Health, 1991
- **MARTIN, ACR**. *Protein Sequence and Structure Analysis of Antibody Variable Domains[J*, 2001
- **LEFRANC, M.P. et al.** *Dev Comp. Immunol.*, 2003, vol. 27, 55-77
- *Front Immunol.*, 16 October 2018, vol. 9, 2278
- *Prot. Sci.*, 2000, vol. 9, 487-496
- Remington's Pharmaceutical Sciences. Mack Publishing Co., 1990
- Remington, The Science and Practice of Pharmacy. Mack Publishing, 2000